(19)

Europäisches
Patentamt
European
Patent Office
Office européen
des brevets

(11)  **EP 3 583 935 A1**

(12)  **EUROPEAN PATENT APPLICATION**

(43) Date of publication:
**25.12.2019  Bulletin 2019/52**

(51) Int Cl.:
**A61K 9/16** (2006.01)

(21) Application number: **19163519.2**

(22) Date of filing: **25.04.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.04.2012  US 201261638073 P
14.03.2013  US 201361783603 P**

(62) Document number(s) of the earlier application(s) in
accordance with Art. 76 EPC:
**13781897.7 / 2 841 057**

(71) Applicant: **SPI Pharma, INC.
Wilmington, DE 19809 (US)**

(72) Inventors:
• **PROPST, Cecil W.
Norton Shores, MI 49441 (US)**

• **MEADOWS, Marc, W
Lot 222, Grand Haven, MI 49417 (US)**
• **TODD, Michael S.
Grand Haven, MI 49417 (US)**

(74) Representative: **Vossius & Partner
Patentanwälte Rechtsanwälte mbB
Siebertstrasse 3
81675 München (DE)**

Remarks:
•Claims filed after the date of receipt of the application
(Rule 68(4) EPC).
•This application was filed on 18-03-2019 as a
divisional application to the application mentioned
under INID code 62.

(54)  **CRYSTALLINE MICROSPHERES AND THE PROCESS FOR MANUFACTURING THE SAME**

(57)    The present invention relates to a composition comprising microspheres, wherein the microspheres have a circularity greater than 0.98, an aspect ratio greater than 0.98, and skeletal density from about 1.4595 g/cc to about 1.4651 g/cc; as well as a pharmaceutical composition comprising the microspheres.

EP 3 583 935 A1

**Description**

CROSS-REFERENCE TO RELATED APPLICATIONS

This application claims priority to U.S. Provisional Application No. 61/638,073 filed April 25, 2012 and U.S. Provisional Application No. 61/783,603 filed March 14, 2013, which are hereby incorporated by reference in their entireties.

FIELD OF THE INVENTION

The present invention relates to a composition comprising a plurality of microspheres, wherein the microspheres are perfectly spherical, and the method of manufacturing the same. The present invention is useful in the manufacture of sustained and modified release active pharmaceutical ingredient (API) microspheres, as a free flowing excipient for mini-tablets and in the manufacture of API carrier dispersions.

BACKGROUND OF THE INVENTION

Many commercial pharmaceutical beads are either reactive or insoluble. Reactive beads such as sucrose/starch beads can cause incompatibility with active substances and loss of active substance due to the presence of reducing sugars. Reaction of moisture in beads made with microcrystalline cellulose, sucrose, starch or cellulose derivatives containing beads can cause incompatibility with active substances and loss of active substance due to the presence of moisture. Loss of API in insoluble beads such as those made with microcrystalline cellulose, starch or cellulose derivatives can result in lack of release of active substance or lower extraction yields from the insoluble materials due to the of insoluble matrixes. Beads made with soluble components such as polyols can be made with very low moisture content (anhydrous) and can be made completely soluble.

Current polyol beads are granulated, thus undissolved polyol particles, primary particles, are "glued" together with a binder solution to make a secondary granular structure. This process makes a surface that is only as smooth and durable as the starting particle size and as the shape will allow. The starting material is not completely liquefied as some remain solid in the granulation route approach and thus transitions are present. Also for very small spheres the contour of the starting particle contributes to a lack of having a smooth crevice-free and bump-free surface, thus lacking perfectly shaped solid spheres. Because the binder contains a solvent, the wet beads must be dried. Bead drying can create internal porosity as well as transition layers of insoluble materials between the undissolved bodies we are calling primary particles. Formation of a wet mass is often done using a granulator, followed by an extruder to form a dense packed pellet and then spinning the pellet on a friction plate into a sphere. Formation can also be done by a powder layering process on a core particle or bead that needs to be large enough to maintain separation in the coating process. This required core and the need to maintain separation restricts the size of the bead that can be made. The layering process starts with seed core upon which insoluble primary particles are deposited and bonded using the binder solution. For effective layering the primary particles must be small enough (<10 $\mu$m for 150 $\mu$m sphere) to be formed into a reasonably smooth surfaced sphere (<30 $\mu$m for 300 $\mu$m sphere). The primary layering particles and the layer application amount must be small enough to prevent porosity and/or moisture from being trapped deep in the sphere. Drying during layering process is critical to balance enough wetness for growth, bead strength and dryness for reduced interior moisture and prevent vacuoles/residual porosity. A water insoluble wicking agent such as MCC aides in the removal of moisture but is insoluble. Final bead size is limited to spheres larger than 100 $\mu$m mean size (10 $\mu$m primary layering particle size) to allow granular shaping and maintain bead separation (preventing twins) during the layering process.

Commercially available beads used as cores as API delivery beads in applications that can survive the temperature/ tumbling conditions of the API coating and layering process are larger than 100 $\mu$m (mean particle diameter). Tablets containing API delivery beads incorporated and compressed into tablets require smaller size beads if bead crushing/ rupturing of the functional coating on coated bead during tablet compression is to be avoided. Tablets containing beads are made typically into swallow tablets to avoid chewing, thus tablet thickness needs to be small to allow ease of swallowing. Beads need to be cushioned during tablet compression to prevent them from being crushed with larger bead requiring more cushioning materials. Larger beads place limitations on the tableting process (slower press speed) and formulation (requires more crushing agents) to create an environment that prevents bead fracturing of larger beads. Smaller beads thus allow for smaller tablets, less cushioning ingredients to be required in tablet formulation as well as in the coating layers and higher dose loading of API.

Excipients for very small mini- tablets (< 3 mm in tablet diameter), require very small excipient particles for fill/tablet weight control. A 1/50 of the diameter of the tablet standard for particle size would require a particle size mean of 60 $\mu$m. Current < 90 $\mu$m particles of microcrystalline cellulose (MCC) (and milled MCC < 90 $\mu$m) are used. These materials are not spherical and thus prone to flow issues causing weight uniformity issues, especially at faster tablet press speeds.

SUMMARY OF THE INVENTION

In one embodiment, the present invention provides for improved microspheres that can be 100% soluble, perfectly spherical, have a uniform surface with limited <2 micron peak to valley roughness, be as small as 2 $\mu$m, be comprised in some embodiments of a single crystal structure with limited or no internal voids, have low hygroscopicity, and low moisture content of less than 1% weight percentage.

In one embodiment, the present invention relates to improved microspheres comprising a core material, wherein the microsphere is perfectly spherical, and the method of manufacturing the same. In some embodiments, the present invention relates to improved microspheres comprising a core material, wherein the microsphere is perfectly spherical and has a moisture content less than 1% by weight. In some embodiments, the present invention relates to improved pharmaceutical microspheres comprising a core material, wherein the microsphere is perfectly spherical, has a smooth surface and has a moisture content less than 1% by weight.

In some embodiments, the present invention relates to improved microspheres comprising a core material, wherein the microsphere is perfectly spherical, has low hygroscopicity, and has a moisture content less than 1% by weight.

BRIEF DESCRIPTION OF THE DRAWINGS

The foregoing summary, as well as the following detailed description of the invention, will be better understood when read in conjunction with the appended drawings. For the purpose of illustration the invention, there are shown in the drawings embodiments which are presently preferred. It should be understood, however, that the invention can be embodied in different forms and thus should not be construed as being limited to the embodiments set forth herein.

Figure 1 is a photomicrograph of exemplary mannitol microspheres of the present invention.
Figure 2 is a SEM photomicrograph (1000x) of exemplary mannitol microspheres of the present invention.
Figure 3 is a close-up (4000x) micrograph of SEM of exemplary mannitol microsphere of the present invention.
Figure 4 is a micrograph of SEM of exemplary mannitol microspheres of the present invention.
Figure 5 is a graph illustrating the moisture content of exemplary mannitol microspheres of the present invention.
Figure 6 (A, B and C) are graphs illustrating the moisture content of powdered, granular and spray-dried mannitols.
Figure 7 is a DSC scan of exemplary mannitol microspheres of the present invention.
Figure 8 (A and B) are micrographs of SEM of sectioned exemplary mannitol microspheres of the present invention.
Figure 9 is a micrograph of SEM of sectioned exemplary mannitol microsphere of the present invention.
Figure 10 is a micrograph of SEM of Celphere CP-102 microcrystalline cellulose beads (Asahi Kasei Corporation, Tokyo, Japan).
Figure 11 is a micrograph of SEM of MCell 400 mannitol beads (Pharmatrans Sanaq AG, Allschwil, Switzerland).
Figure 12 is a micrograph of SEM of Pharm-a-Sphere™ Neutral Pellets (Hanns G. Werner GmbH, Tornesch, Germany).
Figure 13 is a micrograph of SEM of SureSpheres® sugar/starch spheres (Colorcon, West Point, PA).
Figure 14 is micrograph of SEM of Nonpareil-108 mannitol beads (Freund Industrial Co., Japan).
Figure 15 is an image of exemplary mannitol microspheres of the present invention.
Figure 16 is an image of MCell 400 mannitol beads (Pharmatrans Sanaq AG, Allschwil, Switzerland).
Figure 17 is an image of Pharm-a-Sphere™ Neutral Pellets (Hanns G. Werner GmbH, Tornesch, Germany).
Figure 18 is an image of SureSpheres® sugar/starch spheres (Colorcon, West Point, PA).
Figure 19 is an image of Nonpareil-108 mannitol beads (Freund Industrial Co., Ltd., Tokyo, Japan).
Figure 20 is a graph illustrating the circularity of exemplary mannitol microspheres of the present invention in comparison with various commercially available microspheres.
Figure 21 is a graph illustrating the circularity of exemplary mannitol microspheres of the present invention in comparison with various commercially available microspheres.
Figure 22 is a graph illustrating the circularity of exemplary mannitol microspheres of the present invention in comparison with Nonpareil-108 mannitol beads.
Figure 23 is a graph illustrating the aspect ratio of exemplary mannitol microspheres of the present invention in comparison with various commercially available microspheres.
Figure 24 is a graph illustrating the solidity of exemplary mannitol microspheres of the present invention in comparison with various commercially available microspheres.
Figure 25 is a graph illustrating the convexity of exemplary mannitol microspheres of the present invention in comparison with various commercially available microspheres.

DETAILED DESCRIPTION OF INVENTION

The following detailed description is exemplary and explanatory and is intended to provide further explanation of the invention described herein. Other advantages, and novel features will be readily apparent to those skilled in the art from the following detailed description of the invention.

The present invention is described herein using several definitions, as set forth below and throughout the application.

Definitions

The term "about" will be understood by persons of ordinary skill in the art and will vary to some extent on the context in which it is used. If there are uses of the term which are not clear to persons of ordinary skill in the art given the context in which it is used, "about" shall mean up to plus or minus 10% of the particular value.

The terms "solid dosage form," "tablet," and "solid preparation" are used synonymously within the context of the present invention. These terms should be construed to include a compacted or compressed powder composition obtained by compressing or otherwise forming the composition to form a solid having a defined shape.

The aim of the present invention was to overcome the drawbacks of existing commercial beads.

Microsphere refers to a sphere that is from about 1 $\mu$m to about 3 mm. In one embodiment, the present invention relates to a composition comprising a plurality of microspheres. A plurality of microspheres comprises from about 50 to about 20,000 microspheres. In some embodiments, a plurality of microspheres comprises from about 50 to about 15,000 microspheres. In some embodiments, a plurality of microspheres comprises from about 50 to about 10,000 microspheres. In some embodiments, a plurality of microspheres comprises from about 50 to about 5,000 microspheres. In some embodiments, a plurality of microspheres comprises from about 100 to about 20,000 microspheres. In some embodiments, a plurality of microspheres comprises from about 100 to about 15,000 microspheres. In some embodiments, a plurality of microspheres comprises from about 100 to about 10,000 microspheres. In some embodiments, a plurality of microspheres comprises from about 100 to about 5,000 microspheres. In some embodiments, a plurality of microspheres comprises from about 100 to about 1,000 microspheres. In some embodiments, a plurality of microspheres comprises from about 500 to about 20,000 microspheres. In some embodiments, a plurality of microspheres comprises from about 500 to about 15,000 microspheres. In some embodiments, a plurality of microspheres comprises from about 500 to about 10,000 microspheres. In some embodiments, a plurality of microspheres comprises from about 500 to about 5,000 microspheres. In some embodiments, a plurality of microspheres comprises from about 500 to about 1,000 microspheres. In some embodiments, a plurality of microspheres comprises greater than about 50 microspheres. In some embodiments, a plurality of microspheres comprises greater than about 100 microspheres. In some embodiments, a plurality of microspheres comprises greater than about 200 microspheres. In some embodiments, a plurality of microspheres comprises greater than about 300 microspheres. In some embodiments, a plurality of microspheres comprises greater than about 400 microspheres. In some embodiments, a plurality of microspheres comprises greater than about 500 microspheres. In some embodiments, a plurality of microspheres comprises greater than about 750 microspheres. In some embodiments, a plurality of microspheres comprises greater than about 1000 microspheres. In some embodiments, a plurality of microspheres comprises greater than about 1250 microspheres. In some embodiments, a plurality of microspheres comprises greater than about 1500 microspheres. In some embodiments, a plurality of microspheres comprises greater than about 1750 microspheres. In some embodiments, a plurality of microspheres comprises greater than about 2000 microspheres. In some embodiments, a plurality of microspheres comprises greater than about 2500 microspheres. In some embodiments, a plurality of microspheres comprises greater than about 3000 microspheres. In some embodiments, a plurality of microspheres comprises greater than about 3500 microspheres. In some embodiments, a plurality of microspheres comprises greater than about 4000 microspheres. In some embodiments, a plurality of microspheres comprises greater than about 4500 microspheres. In some embodiments, a plurality of microspheres comprises greater than about 5000 microspheres. In some embodiments, a plurality of microspheres comprises greater than about 7500 microspheres. In some embodiments, a plurality of microspheres comprises greater than about 10,000 microspheres. In some embodiments, a plurality of microspheres comprises greater than about 15,000 microspheres.

In some embodiments, the present invention relates to a composition comprising a plurality of microspheres, wherein the microspheres have perfect sphericity. "Perfect sphericity" or "perfectly spherical" means a circularity as measured by imaging microscopy of greater than about 0.90, and an aspect ratio of less than about 1.0. In some embodiments, a composition comprises a plurality of microspheres, wherein the microspheres have a circularity greater than about 0.91. In some embodiments, a composition comprises a plurality of microspheres, wherein the microspheres have a circularity greater than about 0.92. In some embodiments, a composition comprises a plurality of microspheres, wherein the microspheres have a circularity greater than about 0.93. In some embodiments, a composition comprises a plurality of microspheres, wherein the microspheres have a circularity greater than about 0.94. In some embodiments, a composition comprises a plurality of microspheres, wherein the microspheres have a circularity greater than about 0.95. In some embodiments, a composition comprises a plurality of microspheres, wherein the microspheres have a circularity greater than about 0.96. In some embodiments, a composition comprises a plurality of microspheres, wherein the microspheres have a circularity greater than about 0.97. In some embodiments, a composition comprises a plurality of microspheres, wherein the microspheres have a circularity greater than about 0.98. In some embodiments, a composition comprises a plurality of microspheres, wherein the microspheres have a circularity greater than about 0.99. In some embodiments, a composition comprises a plurality of microspheres, wherein at least about 40% of microspheres have a circularity greater than about 0.99. In some embodiments, a composition comprises a plurality of microspheres, wherein at least about 50% of microspheres have a circularity greater than about 0.99. In some embodiments, a composition comprises a plurality of microspheres, wherein at least about 60% of microspheres have a circularity greater than about 0.99. In some embodiments, a composition comprises a plurality of microspheres, wherein at least about 70% of microspheres have a circularity greater than about 0.99. In some embodiments, a composition comprises a plurality of microspheres, wherein about 20% or less of microspheres have a circularity greater than about 0.98. Circularity is calculated in accordance with International Organization for Standardization (ISO) 9276-6 (2008).

In some embodiments, a composition comprises a plurality of microspheres, wherein the microspheres have an aspect ratio of about 0.90. In some embodiments, a composition comprises a plurality of microspheres, wherein the microspheres have an aspect ratio of about 0.91. In some embodiments, a composition comprises a plurality of microspheres, wherein the microspheres have an aspect ratio of about 0.92. In some embodiments, a composition comprises a plurality of microspheres, wherein the microspheres have an aspect ratio of about 0.93. In some embodiments, a composition comprises a plurality of microspheres, wherein the microspheres have an aspect ratio of about 0.94. In some embodiments, a composition comprises a plurality of microspheres, wherein the microspheres have an aspect ratio of about 0.95. In some embodiments, a composition comprises a plurality of microspheres, wherein the microspheres have an aspect ratio of about 0.96. In some embodiments, a composition comprises a plurality of microspheres, wherein the microspheres have an aspect ratio of about 0.97. In some embodiments, a composition comprises a plurality of microspheres, wherein the microspheres have an aspect ratio of about 0.98. In some embodiments, a composition comprises a plurality of microspheres, wherein the microspheres have an aspect ratio of about 0.99. In some embodiments, a composition comprises a plurality of microspheres, wherein the microspheres have an aspect ratio of about 1.0. In some embodiments, a composition comprises a plurality of microspheres, wherein the microspheres have an aspect ratio of about 0.90 or greater. In some embodiments, a composition comprises a plurality of microspheres, wherein the microspheres have an aspect ratio of about 0.91 or greater. In some embodiments, a composition comprises a plurality of microspheres, wherein the microspheres have an aspect ratio of about 0.92 or greater. In some embodiments, a composition comprises a plurality of microspheres, wherein the microspheres have an aspect ratio of about 0.93 or greater. In some embodiments, a composition comprises a plurality of microspheres, wherein the microspheres have an aspect ratio of about 0.94 or greater. In some embodiments, a composition comprises a plurality of microspheres, wherein the microspheres have an aspect ratio of about 0.95 or greater. In some embodiments, a composition comprises a plurality of microspheres, wherein the microspheres have an aspect ratio of about 0.96 or greater. In some embodiments, a composition comprises a plurality of microspheres, wherein the microspheres have an aspect ratio of about 0.97 or greater. In some embodiments, a composition comprises a plurality of microspheres, wherein the microspheres have an aspect ratio of about 0.98 or greater. In some embodiments, a composition comprises a plurality of microspheres, wherein the microspheres have an aspect ratio of about 0.99 or greater. In some embodiments, a composition comprises a plurality of microspheres, wherein at least about 20% of microspheres have an aspect ratio greater than about 0.99. In some embodiments, a composition comprises a plurality of microspheres, wherein at least about 30% of microspheres have an aspect ratio greater than about 0.99. In some embodiments, a composition comprises a plurality of microspheres, wherein at least about 40% of microspheres have an aspect ratio greater than about 0.99. In some embodiments, a composition comprises a plurality of microspheres, wherein at least about 50% of microspheres have an aspect ratio greater than about 0.99. In some embodiments, a composition comprises a plurality of microspheres, wherein at least about 60% of microspheres have an aspect ratio greater than about 0.99. Aspect Ratio is calculated in accordance with International Organization for Standardization (ISO) 9276-6 (2008).

In some embodiments of the present invention, a composition comprises a plurality of microspheres, wherein the microspheres have a convexity of about 0.99. In some embodiments, a composition comprises a plurality of microspheres, wherein at least about 30% of the microspheres have a convexity of about 0.99. In some embodiments, a composition comprises a plurality of microspheres, wherein at least about 40% of the microspheres have a convexity of about 0.99. In some embodiments, a composition comprises a plurality of microspheres, wherein at least about 50% of the microspheres have a convexity of about 0.99. In some embodiments, a composition comprises a plurality of microspheres, wherein at least about 60% of the microspheres have a convexity of about 0.99. In some embodiments, a composition comprises a plurality of microspheres, wherein at least about 70% of the microspheres have a convexity of about 0.99. In some embodiments, a composition comprises a plurality of microspheres, wherein at least about 80% of the microspheres have a convexity of about 0.99. In some embodiments, a composition comprises a plurality of microspheres, wherein about 30% or more of the microspheres have a convexity of about 0.99. In some embodiments, a composition comprises a plurality of microspheres, wherein about 40% or more of the microspheres have a convexity of about 0.99. In some embodiments, a composition comprises a plurality of microspheres, wherein about 50% or more of the microspheres have a convexity of about 0.99. In some embodiments, a composition comprises a plurality of microspheres, wherein about 60% or more of the microspheres have a convexity of about 0.99. In some embodiments, a composition comprises a plurality of microspheres, wherein about 70% or more of the microspheres have a convexity of about 0.99. In some embodiments, a composition comprises a plurality of microspheres, wherein about 80% or more of the microspheres have a convexity of about 0.99. Convexity Ratio is calculated in accordance with International Organization for Standardization (ISO) 9276-6 (2008).

In some embodiments of the present invention, a composition comprises a plurality of microspheres, wherein the microspheres have a solidity of about 0.99. In some embodiments, a composition comprises a plurality of microspheres, wherein at least about 30% of the microspheres have a solidity of about 0.99. In some embodiments, a composition comprises a plurality of microspheres, wherein at least about 40% of the microspheres have a solidity of about 0.99. In some embodiments, a composition comprises a plurality of microspheres, wherein at least about 50% of the microspheres have a solidity of about 0.99. In some embodiments, a composition comprises a plurality of microspheres, wherein at least about 60% of the microspheres have a solidity of about 0.99. In some embodiments, a composition comprises a plurality of microspheres, wherein at least about 70% of the microspheres have a solidity of about 0.99. In some embodiments, a composition comprises a plurality of microspheres, wherein at least about 80% of the microspheres have a solidity of about 0.99. In some embodiments, a composition comprises a plurality of microspheres, wherein at least about 90% of the microspheres have a solidity of about 0.99. In some embodiments, a composition comprises a plurality of microspheres, wherein about 30% or more of the microspheres have a solidity of about 0.99. In some embodiments, a composition comprises a plurality of microspheres, wherein about 40% or more of the microspheres have a solidity of about 0.99. In some embodiments, a composition comprises a plurality of microspheres, wherein about 50% or more of the microspheres have a solidity of about 0.99. In some embodiments, a composition comprises a plurality of microspheres, wherein about 60% or more of the microspheres have a solidity of about 0.99. In some embodiments, a composition comprises a plurality of microspheres, wherein about 70% or more of the microspheres have a solidity of about 0.99. In some embodiments, a composition comprises a plurality of microspheres, wherein about 80% or more of the microspheres have a solidity of about 0.99. In some embodiments, a composition comprises a plurality of microspheres, wherein about 90% or more of the microspheres have a solidity of about 0.99. Solidity Ratio is calculated in accordance with International Organization for Standardization (ISO) 9276-6 (2008).

In some embodiments of the present invention, a composition comprises a plurality of microspheres, wherein the microspheres have a mean particle size from about 2 $\mu$m to about 3000 $\mu$m in diameter. In some embodiments of the present invention, a composition comprises a plurality of microspheres, wherein the microspheres have a mean particle size from about 2 $\mu$m to about 10 $\mu$m in diameter. In some embodiments of the present invention, a composition comprises a plurality of microspheres, wherein the microspheres have a mean particle size from about 10 $\mu$m to about 20 $\mu$m in diameter. In some embodiments of the present invention, a composition comprises a plurality of microspheres, wherein the microspheres have a mean particle size from about 10 $\mu$m to about 500 $\mu$m in diameter. This mean particle size d(0.5) can be anywhere within this range based on process conditions chosen. In one embodiment of the present invention, a composition comprises a plurality of microspheres, wherein the microspheres have a particle size distribution (d(0.9)/d(0.1)) of about 2.8 or less. In another embodiment of the present invention, a composition comprises a plurality of microspheres, wherein the microspheres have a particle size distribution of about 2.7 or less. In another embodiment of the present invention, a composition comprises a plurality of microspheres, wherein the microspheres have a particle size distribution of about 2.4 or less. In another embodiment of the present invention, a composition comprises a plurality of microspheres, wherein the microspheres have a particle size distribution of about 2.3 or less. In another embodiment of the present invention, a composition comprises a plurality of microspheres, wherein the microspheres have a particle size distribution of about 2.2 or less. In another embodiment of the present invention, a composition comprises a plurality of microspheres, wherein the microspheres have a particle size distribution of about 2.1 or less. In another embodiment of the present invention, a composition comprises a plurality of microspheres, wherein the microspheres have a mean particle size distribution of about 2.0 or less. In another embodiment of the present invention, a composition comprises a plurality of microspheres, wherein the microspheres have a particle size distribution of about 1.9 or less. In another embodiment of the present invention, a composition comprises a plurality of microspheres, wherein the microspheres have a particle size distribution of about 1.8 or less. In another embodiment of the present invention, a composition comprises a plurality of microspheres, wherein the microspheres have a particle size distribution of about 1.7 or less. In another embodiment of the present invention, a composition comprises a plurality of microspheres, wherein the microspheres have a particle size distribution of about 1.6 or less. In another embodiment of the present invention, a composition comprises a plurality of microspheres, wherein the microspheres have a particle size distribution of about 1.5 or less. In another embodiment of the present invention, a composition comprises a plurality of microspheres, wherein the microspheres have a particle size distribution of about 1.4 or less. In another embodiment of the present invention, a composition comprises a plurality of microspheres, wherein the microspheres have a particle size distribution of about 1.3 or less. In another embodiment of the present invention, a composition comprises a plurality of microspheres, wherein the microspheres have a particle size distribution of about 1.2 or less. In some embodiments, the present invention provides for microspheres with a narrow particle size distribution without a first microsphere size separation step. In some embodiments, the present invention provides for microspheres with a narrow particle size distribution prior to a first microsphere size separation step.

In some embodiments, the perfect sphericity, perfect solidity, narrow particle density and lack of pores of the microspheres of the present invention create the ability to use size selection to narrowly control the effective surface area (ESA) for coating the microspheres per mass. The effective surface area is the surface at the base of the functional film or coating whose thickness for performance is fixed at a minimum and a maximum. Normal for functional films or coating, the thickness required is a film or coating of 10 $\mu$m or more. If substantially all of the surface of the microsphere is part of the start of the functional thickness layering it is as if the coating or film is being applied to a flat surface. Thus the build of thickness is uniform and reproducible. Coating lost into pores or needed over risers is not lost if the microsphere has both a high solidity and a high convexity value. The surface area for the weight of beads used in the coating batch can thus be related directly to bead size and bead size frequency even down to 10 $\mu$m beads. Factors of shape, bead density, and effective surface lost into crevices and pores and surface distortions from risers are no longer factors that affect the relationship of size selection to the effective surface per batch to be coated. Also particle flow of a circular microsphere at 10 $\mu$m is maintained as particles are spherical and particle to particle contact is at points of contact and thus minimal surface area involved. Also minimal moisture < 0.2% generates a very small surface free energy at contact points. In some embodiments, microspheres of the present invention also can be made static free as the process is a crystallization process, even at the 10 $\mu$m level. Maintaining separation is extremely important to prevent agglomeration during coating and use. Aerodynamics is also uniform based on the microspheres having uniform shape and density. Submicron crystal ridges exist even on the 10 $\mu$m microsphere thus even though the microsphere appears smooth the surface can be attached to with coating materials.

In some embodiments, the present invention provides for the manufacture of microspheres that have a standardizable surface area, due to their perfectly spherical shape and their lack of internal porosity. In some embodiments of the present invention, the microsphere lacks internal porosity. In some embodiments, the microsphere lacks internal voids. A void is defined as an area in the bead that is not open to the surface and thus not a pore. This open area normally filled with air causes the density of the particle to be lowered when present. If present, these pores create particle density variability as their presence is usually not uniform. Thus in process using sonic nozzles or using a size selection process a specific narrow range of surface area/ process weight used will allow coating a much more exact surface area and the variability of film thickness controlled. Current commercial beads are made and available in size ranges. In some embodiments, the present invention provides for the manufacture and/or sorting of microspheres to provide microspheres of a standard surface and much more narrowed surface area range per weight of beads used per batch for coating process. This provides for a uniformly coated microsphere and narrows the distribution of coating film thicknesses from batch to batch.

In some embodiments of the present invention, a composition comprises a plurality of microspheres, wherein the microspheres have a smooth surface. A smooth surface with lack of bumps is essential for uniformity in the coating thickness of an API and decreasing the risk of pin hole formations in the coating film. However small ridges in the microsphere surface aide in the adherence of binder or coating solution to the surface of the microsphere.. In one embodiment, a composition comprises a plurality of microspheres, wherein the microspheres have a surface with ridges of about 4 $\mu$m or less in height. In one embodiment, a composition comprises a plurality of microspheres, wherein the microspheres have a surface with ridges of about 3 $\mu$m or less in height. In one embodiment, a composition comprises a plurality of microspheres, wherein the microspheres have a surface with ridges of about 2 $\mu$m or less in height. In one embodiment, a composition comprises a plurality of microspheres, wherein the microspheres has a surface with ridges of about 1 $\mu$m or less in height. In one embodiment, a microsphere has a surface with ridges of about 0.5 $\mu$m or less in height. In one embodiment, a microsphere has a surface with ridges of less than about 4 $\mu$m in height. In one embodiment, a microsphere has a surface with ridges of less than about 3 $\mu$m in height. In one embodiment, a microsphere has a surface with ridges of less than about 2 $\mu$m in height. In one embodiment, a microsphere has a surface with ridges of <1 $\mu$m in height. In one embodiment, a microsphere has a surface with ridges of less than about 0.9 $\mu$m in height. In one embodiment, a microsphere has a surface with ridges of less than about 0.8 $\mu$m in height. In one embodiment, a microsphere has a surface with ridges of less than about 0.7 $\mu$m in height. In one embodiment, a microsphere has a surface with ridges of less than about 0.6 $\mu$m in height. In one embodiment, a microsphere has a surface with ridges of less than about 0.5 $\mu$m in height. In one embodiment, a microsphere has a surface with ridges of less than about 0.4 $\mu$m in height. In one embodiment, a microsphere has a surface with ridges of less than about 0.3 $\mu$m in height. In one embodiment, a microsphere has a surface with ridges of less than about 0.2 $\mu$m in height. In one embodiment, a microsphere has a surface with ridges of less than about 0.1 $\mu$m in height. In one embodiment, a microsphere has a surface with ridges of less than about 0.05 $\mu$m in height.

In some embodiments, a composition comprising a plurality of microspheres does not have any ridges that

exceed about 1 μm in height. In some embodiments, a composition comprising a plurality of microspheres does not have any ridges that exceed about 0.9 μm in height. In some embodiments, a composition comprising a plurality of microspheres does not have any ridges that exceed about 0.8 μm in height. In some embodiments, a composition comprising a plurality of microspheres does not have any ridges that exceed about 0.7 μm in height. In some embodiments, a composition comprising a plurality of microspheres does not have any ridges that exceed about 0.6 μm in height. In some embodiments, a composition comprising a plurality of microspheres does not have any ridges that exceed about 0.5 μm in height. In some embodiments, a composition comprising a plurality of microspheres does not have any ridges that exceed about 0.4 μm in height. In some embodiments, a composition comprising a plurality of microspheres does not have any ridges that exceed about 0.3 μm in height. In some embodiments, a composition comprising a plurality of microspheres does not have any ridges that exceed about 0.2 μm in height. In some embodiments, a composition comprising a plurality of microspheres does not have any ridges that exceed about 0.1 μm in height. In some embodiments, a composition comprising a plurality of microspheres does not have any ridges that exceed about 0.05 μm in height.

In some embodiments, a composition comprises a plurality of microspheres, wherein about 80% of the microspheres do not have any ridges that exceed about 1 μm in height. In some embodiments, a composition comprises a plurality of microspheres, wherein about 85% of the microspheres do not have any ridges that exceed about 1 μm in height. In some embodiments, a composition comprises a plurality of microspheres, wherein about 90% of the microspheres do not have any ridges that exceed about 1 μm in height. In some embodiments, a composition comprises a plurality of microspheres, wherein about 95% of the microspheres do not have any ridges that exceed about 1 μm in height. In some embodiments, a composition comprises a plurality of microspheres, wherein about 99% of the microspheres do not have any ridges that exceed about 1 μm in height. In some embodiments, a composition comprises a plurality of microspheres, wherein about 80% of the microspheres do not have any ridges that exceed about 0.9 μm in height. In some embodiments, a composition comprises a plurality of microspheres, wherein about 85% of the microspheres do not have any ridges that exceed about 0.9 μm in height. In some embodiments, a composition comprises a plurality of microspheres, wherein about 90% of the microspheres do not have any ridges that exceed about 0.9 μm in height. In some embodiments, a composition comprises a plurality of microspheres, wherein about 95% of the microspheres do not have any ridges that exceed about 0.9 μm in height. In some embodiments, a composition comprises a plurality of microspheres, wherein about 99% of the microspheres do not have any ridges that exceed about 0.9 μm in height. In some embodiments, a composition comprises a plurality of microspheres, wherein about 80% of the microspheres do not have any ridges that exceed about 0.8 μm in height. In some embodiments, a composition comprises a plurality of microspheres, wherein about 85% of the microspheres do not have any ridges that exceed about 0.8 μm in height. In some embodiments, a composition comprises a plurality of microspheres, wherein about 90% of the microspheres do not have any ridges that exceed about 0.8 μm in height. In some embodiments, a composition comprises a plurality of microspheres, wherein about 95% of the microspheres do not have any ridges that exceed about 0.8 μm in height. In some embodiments, a composition comprises a plurality of microspheres, wherein about 99% of the microspheres do not have any ridges that exceed about 0.8 μm in height. In some embodiments, a composition comprises a plurality of microspheres, wherein about 80% of the microspheres do not have any ridges that exceed about 0.7 μm in height. In some embodiments, a composition comprises a plurality of microspheres, wherein about 85% of the microspheres do not have any ridges that exceed about 0.7 μm in height. In some embodiments, a composition comprises a plurality of microspheres, wherein about 90% of the microspheres do not have any ridges that exceed about 0.7 μm in height. In some embodiments, a composition comprises a plurality of microspheres, wherein about 95% of the microspheres do not have any ridges that exceed about 0.7 μm in height. In some embodiments, a composition comprises a plurality of microspheres, wherein about 99% of the microspheres do not have any ridges that exceed about 0.7 μm in height. In some embodiments, a composition comprises a plurality of microspheres, wherein about 80% of the microspheres do not have any ridges that exceed about 0.6 μm in height. In some embodiments, a composition comprises a plurality of microspheres, wherein about 85% of the microspheres do not have any ridges that exceed about 0.6 μm in height. In some embodiments, a composition comprises a plurality of microspheres, wherein about 90% of the microspheres do not have any ridges that exceed about 0.6 μm in height. In some embodiments, a composition comprises a plurality of microspheres, wherein about 95% of the microspheres do not have any ridges that exceed about 0.6 μm in height. In some embodiments, a composition comprises a plurality of microspheres, wherein about 99% of the microspheres do not have any ridges that exceed about 0.6 μm in height. In some embodiments, a composition comprises a plurality of microspheres, wherein about 80% of the microspheres do not have any ridges that exceed about 0.5 μm in height. In some embodiments, a composition comprises a plurality of microspheres, wherein about 85% of the microspheres do not have any ridges that exceed about 0.5 μm in height. In some embodiments, a composition comprises a plurality of microspheres, wherein about 90% of the microspheres do not have any ridges that exceed about 0.5 μm in height. In some embodiments, a composition comprises a plurality of microspheres, wherein about 95% of the microspheres do not have any ridges that exceed about 0.5 μm in height. In some embodiments, a composition comprises a plurality of microspheres, wherein about 99% of the microspheres do not have any ridges that exceed about 0.5 μm in height. In some embodiments, a composition comprises a plurality of microspheres, wherein about 80% of the microspheres do not have any ridges that exceed about 0.4 μm in height. In some embodiments, a composition comprises a plurality of microspheres, wherein about 85% of the microspheres do not have any ridges that exceed about 0.4 μm in height. In some embodiments, a composition comprises a plurality of microspheres, wherein about 90% of the microspheres do not have any ridges that exceed about 0.4 μm in height. In some embodiments, a composition comprises a plurality of microspheres, wherein about 95% of the microspheres do not have any ridges that exceed about 0.4 μm in height. In some embodiments, a composition comprises a plurality of microspheres, wherein about 99% of the microspheres do not have any ridges that exceed about 0.4 μm in height. In some embodiments, a composition comprises a plurality of microspheres, wherein about 80% of the microspheres do not have any ridges that exceed about 0.3 μm in height. In some embodiments, a composition comprises a plurality of microspheres, wherein about 85% of the microspheres do not have any ridges that exceed about 0.3 μm in height. In some embodiments, a composition comprises a plurality of microspheres, wherein about 90% of the microspheres do not have any ridges that exceed about 0.3 μm in height. In some embodiments, a composition comprises a plurality of microspheres, wherein about 95% of the microspheres do not have any ridges that exceed about 0.3 μm in height. In some embodiments, a composition comprises a plurality of microspheres, wherein about 99% of the microspheres do not have any ridges that exceed about 0.3 μm in height. In some embodiments, a composition comprises a plurality of microspheres, wherein about 80% of the microspheres do not have any ridges that exceed about 0.2 μm in height. In some embodiments, a composition comprises a plurality of microspheres, wherein about 85% of the microspheres do not have any ridges that exceed about 0.2 μm in height. In some embodiments, a composition comprises a plurality of microspheres, wherein about 90% of the microspheres do not have any ridges that exceed about 0.2 μm in height. In some embodiments, a composition comprises a plurality of microspheres, wherein about 95% of the microspheres do not have any ridges that exceed about 0.2 μm in height. In some embodiments, a composition comprises a plurality of microspheres, wherein about 99% of the microspheres do not have any ridges that exceed about 0.2 μm in height. In some embodiments, a composition comprises a plurality of microspheres, wherein about 80% of the microspheres do not have any ridges that exceed about 0.1 μm in height. In some embodiments, a composition comprises a plurality of microspheres, wherein about 85% of the microspheres do not have any ridges that exceed about 0.1 μm in height. In some embodiments, a composition comprises a plurality of microspheres, wherein about 90% of the microspheres do not have any ridges that exceed about 0.1 μm in height. In some embodiments, a composition comprises a plurality of microspheres, wherein about 95% of the microspheres do not have any ridges that exceed about 0.1 μm in height. In some embodiments, a composition comprises a plurality of microspheres, wherein about 99% of the microspheres do not have any ridges that exceed about 0.1 μm in height. In some embodiments, a composition comprises a plurality of microspheres, wherein about 80% of the microspheres do not have any ridges that exceed about 0.05 μm in height. In some embodiments, a composition comprises a plurality of microspheres, wherein about 85% of the microspheres do not have any ridges that exceed about 0.05 μm in height. In some embodiments, a composition comprises a plurality of microspheres, wherein about 90% of the microspheres do not have any ridges that exceed about 0.05 μm in height. In some embodiments, a composition comprises a plurality of microspheres, wherein about 95% of the microspheres do not have any ridges that exceed about 0.05 μm in height. In some embodiments, a composition comprises a plurality of microspheres, wherein about 99% of the microspheres do not have any ridges that exceed about 0.05 μm in height.

In some embodiments, a composition comprises a plurality of microspheres, wherein the surface of the microsphere is comprised of flat, crystal plates. In some embodiments, a composition comprises a plurality of microspheres, wherein the surface of the microsphere is comprised of flat, crystal plates that include ridges at one or more portions of a periphery of the plates. In one embodiment, the ridges extend radially from the periphery of the plate away from the center or core of the microsphere. In one embodiment, the ridges extend radially away from the surface to the center or core of the microsphere. In some embodiments, the crystal plates with ridges form shallow ridges of less than about 2 μm in height. This allows a film polymer to grip to during the early stages of coating and creates a surface with minimum loss of film material. In some embodiments, the formation of the flat crystal plates is crystals extending to the surface of the microsphere in stacked flat crystals forms of either that from growth terminations as a stacked group of thin crystals in bundles, each bundle creating a surface plate. In this embodiment, surface ridges are created by crystal plates on the surface that are less than about 1 μm in height. In another embodiment, the flat crystal plates on the microsphere surface form as crystalization layers in an onion layer growth pattern. In some embodiments, the surface ridges occur at a frequency greater than about one per μm distance along the surface of a microsphere. In some embodiments, the surface ridges occur at a frequency greater than about two per μm distance along the surface of a microsphere. In some embodiments, the surface ridges occur at a frequency greater than about three per μm distance along the surface of a microsphere. In some embodiments, the surface ridges occur at a frequency greater than about four per μm distance along the surface of a microsphere. In some embodiments, the surface ridges occur at a frequency greater than about five per μm distance along the surface of a microsphere. In some embodiments, the surface ridges occur at a frequency of about one per μm distance along the surface of a microsphere. In some embodiments, the surface ridges occur at a frequency of about two per μm distance along the surface of a microsphere. In some embodiments, the surface ridges occur at a frequency of about three per μm distance along the surface of a microsphere. In some embodiments, the surface ridges occur at a frequency of about four per μm distance along the surface of a microsphere. In some embodiments, the surface ridges occur at a frequency of about five per μm distance along the surface of a microsphere. In some embodiments, the surface ridges occur at a frequency of about one to about five per μm distance along the surface of a microsphere. In some embodiments, the surface ridges occur at a frequency of about two to about five per μm distance along the surface of a microsphere. In some embodiments, the surface ridges occur at a frequency of about three to about five per μm distance along the surface of a microsphere. In some embodiments, the surface ridges occur at a frequency of about four to about five per μm distance along the surface of a microsphere.

In some embodiments, the surface ridges occur at a frequency of less than about one per μm distance along the surface of a microsphere. In some embodiments, the surface ridges occur at a frequency of less than about two per μm distance along the surface of a microsphere. In some embodiments, the surface ridges occur at a frequency of less than about three per μm distance along the surface of a microsphere. In some embodiments, the surface ridges occur at a frequency of less than about four per μm distance along the surface of a microsphere. In some embodiments, the surface ridges occur at a frequency of less than about five per μm distance along the surface of a microsphere.

In some embodiments, the flat crystal plates on the surface of the microsphere are horizontal to a surface of the microsphere and are formed either during the droplet formation due to spinning orientation and/or cooling (surface nucleation orientation) of the droplet to form the solid microsphere. In some embodiments, the flat, crystal plates on the surface of the microsphere is formed by the surface of the spinning disc. In some embodiments, the flat, crystal plates on the microsphere surface are horizontal to a surface of the microsphere. This may be based on spin roll. In some embodiments, the flat, crystal plates on the microsphere surface is formed as a molecular orientation on the disc. In some embodiments, the flat, crystal plates on the microsphere surface is formed from the cooling process. In some embodiments, the flat, crystal plates on the surface of the microsphere formed by the surface of the spinning disk is adjustable based on the surface of the spin disc temperature. In some embodiments, the flat, crystal plates on the surface of the microsphere formed by the surface of the spinning disk is adjustable based on the spinning speed. In some embodiments, the flat, crystal plates on the surface of the microsphere formed by the surface of the spinning disk is adjustable based on the surface of the spin disc temperature and the spinning speed. Although the use of a spinning disc is one method of making the microspheres according the inventions described here, the microspheres described herein are not limited to those microspheres resulting from the methods disclosed herein. Indeed, the microspheres according to the inventions described herein can be made by any method that will result in the microspheres according to the inventions described herein.

In contrast, risers, rounded projections from the surface of a microsphere, cause coating thickness variation by generating local spots of thin coating or pin holes. If a functional coat must be 10 μm thick and pin holes need to be avoided then the coating over the riser needs to be 10 μm thick, making the coating in other regions required to be thicker. In some embodiments, the surface of the microspheres of the present invention has uniform ridges which coating materials can grip to hold onto. In some embodiments, these ridges have a less than about 2 μm depth and thus contribute little to coating thickness variability. In some embodiments, a composition comprises a plurality of microspheres, wherein the surface of the microsphere does not have risers.

In some embodiments, a composition comprises a plurality of microspheres, wherein the microsphere has vertical or radially rising, very tightly packed crystal formations that are underneath the surface of the microsphere. In some embodiments, the vertical or radially rising, and very tightly packed crystal formations in the microsphere and the solid center of the microsphere allow the skeletal density of the microsphere to approach the true density reported for alpha mannitol, and allow for a very narrow control of particle density.

In some embodiments of the present invention, a composition comprises a plurality of microspheres, wherein the microspheres have a skeletal density of about 1.4595 to about 1.4651 gm/cc by helium pynometry. In some embodiments, a composition comprises a plurality of microspheres, wherein the microspheres have an average skeletal density of about 1.461 gm/cc. This is compared to the true density by x-ray diffraction of a polyol alpha mannitol crystal form found of 1.468 gm/ml. In some embodiments, this develops a bead porosity of $(1-(1.468 - 1.461)/1.468) * 100 = \sim 0\%$. This crystal lattice density matches the DSC scan for beads showing a match of crystal energy of alpha mannitol and the melting point match to alpha mannitol identifies these beads a solid crystal alpha mannitol forms. In some embodiments, a composition comprises a plurality of microspheres, wherein the microspheres have a skeletal density within about 10 % of the skeletal density of the microspheres' material. In some embodiments, a composition comprises a plurality of microspheres, wherein the microspheres have a skeletal density within about -10 % and +10 % of the skeletal density of the microspheres' material. In some embodiments, a composition comprises a plurality of microspheres, wherein the microspheres have a skeletal density within about -9 % and +9 % of the skeletal density of the microspheres' material. In some embodiments, a composition comprises a plurality of microspheres, wherein the microspheres have a skeletal density within about -8 % and +8 % of the skeletal density of the microspheres' material. In some embodiments, a composition comprises a plurality of microspheres, wherein the microspheres have a skeletal density within about -7 % and +7 % of the skeletal density of the microspheres' material. In some embodiments, a composition comprises a plurality of microspheres, wherein the microspheres have a skeletal density within about -6 % and +6 % of the skeletal density of the microspheres' material. In some embodiments, a composition comprises a plurality of microspheres, wherein the microspheres have a skeletal density within about -5 % and +5 % of the skeletal density of the microspheres' material. In some embodiments, a composition comprises a plurality of microspheres, wherein the microspheres have a skeletal density within about -4 % and +4 % of the skeletal density of the microspheres' material. In some embodiments, a composition comprises a plurality of microspheres, wherein the microspheres have a skeletal density within about -3 % and +3 % of the skeletal density of the microspheres' material. In some embodiments, a composition comprises a plurality of microspheres, wherein the microspheres have a skeletal density within about -2 % and +2 % of the skeletal density of the microspheres' material. In some embodiments, a composition comprises a plurality of microspheres, wherein the microspheres have a skeletal density within about -1 % and +1 % of the skeletal density of the microspheres' material. In some embodiments, a composition comprises a plurality of microspheres, wherein the microspheres have a skeletal density within about -0.9 % and +0.9 % of the skeletal density of the microspheres' material. In some embodiments, a composition comprises a plurality of microspheres, wherein the microspheres have a skeletal density within about -0.8 % and +0.8 % of the skeletal density of the microspheres' material. In some embodiments, a composition comprises a plurality of microspheres, wherein the microspheres have a skeletal density within about -0.7 % and +0.7 % of the skeletal density of the microspheres' material. In some embodiments, a composition comprises a plurality of microspheres, wherein the microspheres have a skeletal density within about -0.6 % and +0.6 % of the skeletal density of the microspheres' material. In some embodiments, a composition comprises a plurality of microspheres, wherein the microspheres have a skeletal density within about -0.5 % and +0.5 % of the skeletal density of the microspheres' material. In some embodiments, a composition comprises a plurality of microspheres, wherein the microspheres have a skeletal density within about -0.4 % and +0.4 % of the skeletal density of the microspheres' material. In some embodiments, a composition comprises a plurality of microspheres, wherein the microspheres have a skeletal density within about -0.3 % and +0.3 % of the skeletal density of the microspheres' material. In some embodiments, a composition comprises a plurality of microspheres, wherein the microspheres have a skeletal density within about -0.2 % and +0.2 % of the skeletal density of the microspheres' material. In some embodiments, a composition comprises a plurality of microspheres, wherein the microspheres have a skeletal density within about -0.1 % and +0.1 % of the skeletal density of the microspheres' material.

In some embodiments of the present invention, a composition comprises a plurality of microspheres, wherein the microspheres have low hygroscopicity. In one embodiment, a composition comprises a plurality of microspheres, wherein the microspheres have a moisture gain of about 0.18% by weight at about 90% relative humidity. In one embodiment, a microsphere has moisture gain of less than about 2.0% by weight at about 90% relative humidity. In one embodiment, a microsphere has moisture gain of less than about 1.9% by weight at about 90% relative humidity. In one embodiment, a microsphere has moisture gain of less than about1.8% by weight at about 90% relative humidity. In one embodiment, a microsphere has moisture gain of less than about 1.7% by weight at about 90% relative humidity. In one embodiment, a microsphere has moisture gain of less than about 1.6% by weight at about 90% relative humidity. In one embodiment, a microsphere has moisture gain of less than about 1.5% by weight at about 90% relative humidity. In one embodiment, a microsphere has moisture gain of less than about1.4% by weight at about 90% relative humidity. In one embodiment, a microsphere has moisture gain of less than about 1.3% by weight at about 90% relative humidity. In one embodiment, a microsphere has moisture gain of less than about 1.2% by weight at about 90% relative humidity. In one embodiment, a microsphere has moisture gain of less than about 1.1% by weight at about 90% relative humidity. In one embodiment, a microsphere has moisture gain of less than about 1.0% by weight at about 90% relative humidity. In one embodiment, a microsphere has moisture gain of less than about 0.9% by weight at about 90% relative humidity. In one embodiment, a microsphere has moisture gain of less than about 0.8% by weight at about 90% relative humidity. In one embodiment, a microsphere has moisture gain of less than about 0.7% by weight at about 90% relative humidity. In one embodiment, a microsphere has moisture gain of less than about 0.6% by weight at about 90% relative humidity. In one embodiment, a microsphere has moisture gain of less than about 0.5% by weight at about 90% relative humidity. In one embodiment, a microsphere has moisture gain of less than about 0.4% by weight at about 90% relative humidity. In one embodiment, a microsphere has moisture gain of less than about 0.3% by weight at about 90% relative humidity. In one embodiment, a microsphere has moisture gain of less than about 0.2% by weight at about 90% relative humidity. In one embodiment, a microsphere has moisture gain of less than about 0.1% by weight at about 90% relative humidity. In one embodiment, a microsphere has moisture gain of less than about 0.05% by weight at about 90% relative humidity. In one embodiment, a microsphere has moisture gain of about 0% by weight at about 90% relative humidity.

In another embodiment, a microsphere has a moisture gain of less than about 1.00% by weight at about 60% relative humidity. In another embodiment, a microsphere has a moisture gain of less than about 0.90% by weight at about 60% relative humidity. In another embodiment, a microsphere has a moisture gain of less than about 0.80% by weight at about 60% relative humidity. In another embodiment, a microsphere has a moisture gain of less than about 0.70% by weight at about 60% relative humidity. In another embodiment, a microsphere has a moisture gain of less than about 0.60% by weight at about 60% relative humidity. In another embodiment, a microsphere has a moisture gain of less than about 0.50% by weight at about 60% relative humidity. In another embodiment, a microsphere has a moisture gain of less than about 0.40% by weight at about 60% relative humidity. In another embodiment, a microsphere has a moisture gain of less than about 0.30% by weight at about 60% relative humidity. In another embodiment, a microsphere has a moisture gain of less than about 0.20% by weight at about 60% relative humidity. In another embodiment, a microsphere has a moisture gain of less than about 0.10% by weight at about 60% relative humidity. In another embodiment, a microsphere has a moisture gain of less than about 0.09% by weight at about 60% relative humidity. In another embodiment, a microsphere has a moisture gain of less than about 0.08% by weight at about 60% relative humidity. In another embodiment, a microsphere has a moisture gain of less than about 0.07% by weight at about 60% relative humidity. In another embodiment, a microsphere has a moisture gain of less than about 0.06% by weight at about 60% relative humidity. In another embodiment, a microsphere has a moisture gain of less than about 0.05% by weight at about 60% relative humidity. In another embodiment, a microsphere has a moisture gain of less than about 0.04% by weight at about 60% relative humidity. In another embodiment, a microsphere has a moisture gain of less than about 0.03% by weight at about 60% relative humidity. In another embodiment, a microsphere has a moisture gain of less than about 0.02% by weight at about 60% relative humidity. In another embodiment, a microsphere has a moisture gain of less than about 0.01% by weight at about 60% relative humidity. In another embodiment, a microsphere has a moisture gain of about 0% by weight at about 60% relative humidity. Hygroscopicity is measured by Dynamic Vapor Sorption (DVS).

In some embodiments of the present invention, a microsphere has a moisture content of about 2.0% by weight or less. In one embodiment, a microsphere has a moisture content of about 1.9% by weight or less. In one embodiment, a microsphere has a moisture content of about 1.8% by weight or less. In one embodiment, a microsphere has a moisture content of about 1.7% by weight or less. In one embodiment, a microsphere has a moisture content of about 1.6% by weight or less. In one embodiment, a microsphere has a moisture content of about 1.5% by weight or less. In one embodiment, a microsphere has a moisture content of about 1.4% by weight or less. In one embodiment, a microsphere has a moisture content of about 1.3% by weight or less. In one embodiment, a microsphere has a moisture content of about 1.2% by weight or less. In one embodiment, a microsphere has a moisture content of about 1.1% by weight or less. In one embodiment, a microsphere has a moisture content of about 1.0% by weight or less. In one embodiment, a microsphere has a moisture content of about 0.9% by weight or less. In one embodiment, a microsphere has a moisture content of about 0.8% by weight or less. In one embodiment, a microsphere has a moisture content of about 0.7% by weight or less. In one embodiment, a microsphere has a moisture content of about 0.6% by weight or less. In one embodiment, a microsphere has a moisture content of about 0.5% by weight or less. In one embodiment, a microsphere has a moisture content of about 0.4% by weight or less. In one embodiment, a microsphere has a moisture content of about 0.3% by weight or less. In one embodiment, a microsphere has a moisture content of about 0.2% by weight or less. In one embodiment, a microsphere has a moisture content of about 0.1% by weight or less. In one embodiment, a microsphere has a moisture content of about 0.09% by weight or less. In one embodiment, a microsphere has a moisture content of about 0.08% by weight or less. In one embodiment, a microsphere has a moisture content of about 0.07% by weight or less. In one embodiment, a microsphere has a moisture content of about 0.06% by weight or less. In one embodiment, a microsphere has a moisture content of about 0.05% by weight or less. In one embodiment, a microsphere has a moisture content of about 0.04% by weight or less. In one embodiment, a microsphere has a moisture content of about 0.03% by weight or less. In one embodiment, a microsphere has a moisture content of about 0.02% by weight or less. Moisture content is measured by loss on drying using Karl Fisher method. Hygroscopicity and moisture content are important characteristics of microspheres or beads as they may affect changes in active pharmaceutical ingredients (APIs), such as amorphous APIs, APIs sensitive to hydration, micronized and freeze-dried APIs that are sensitive to moisture.

In some embodiments of the present invention, a composition comprises a plurality of microspheres, wherein the microspheres have a dissolution of less than about 2%. In some embodiments of the present invention, a composition comprises a plurality of microspheres, wherein the microspheres have a dissolution of less than about 1.5%. In some embodiments of the present invention, a composition comprises a plurality of microspheres, wherein the microspheres have a dissolution of less than about 1%. In some embodiments of the present invention, a composition comprises a plurality of microspheres, wherein the microspheres have a dissolution of less than about 0.5%. Dissolution is determined as set forth herein in Example 2.

In some embodiments of the present invention, a composition comprises a plurality of microspheres, wherein the microspheres have an oil adsorption capacity of less than about 5%. In some embodiments of the present invention, a composition comprises a plurality of microspheres, wherein the microspheres have an oil adsorption capacity of less than about 4%. In some embodiments of the present invention, a composition comprises a plurality of microspheres, wherein the microspheres have an oil adsorption capacity of less than about 3%. In some embodiments of the present invention, a composition comprises a plurality of microspheres, wherein the microspheres have an oil adsorption capacity of less than about 2%. In some embodiments of the present invention, a composition comprises a plurality of microspheres, wherein the microspheres have an oil adsorption capacity of less than about 1%. In some embodiments of the present invention, a composition comprises a plurality of microspheres, wherein the microspheres have an oil adsorption capacity of less than about 0.9%. In some embodiments of the present invention, a composition comprises a plurality of microspheres, wherein the microspheres have an oil adsorption capacity of less than about 0.8%. In some embodiments of the present invention, a composition comprises a plurality of microspheres, wherein the microspheres have an oil adsorption capacity of less than about 0.7%. In some embodiments of the present invention, a composition comprises a plurality of microspheres, wherein the microspheres have an oil adsorption capacity of less than about 0.6%. In some embodiments of the present invention, a composition comprises a plurality of microspheres, wherein the microspheres have an oil adsorption capacity of less than about 0.5%. In some embodiments of the present invention, a composition comprises a plurality of microspheres, wherein the microspheres have an oil adsorption capacity of less than about 0.4%. In some embodiments of the present invention, a composition comprises a plurality of microspheres, wherein the microspheres have an oil adsorption capacity of less than about 0.3%. In some embodiments of the present invention, a composition comprises a plurality of microspheres, wherein the microspheres have an oil adsorption capacity of less than about 0.2%. In some embodiments of the present invention, a composition comprises a plurality of microspheres, wherein the microspheres have an oil adsorption capacity of less than about 0.1%. Oil Adsorption Capacity is determined as set forth herein in Example 2.

In some embodiments of the present invention, a composition comprises a plurality of microspheres, wherein the microspheres have an actual to predicted surface area ratio of about 5 or less. In some embodiments of the present invention, a composition comprises a plurality of microspheres, wherein the microspheres have an actual to predicted surface area ratio of about 4 or less. In some embodiments of the present invention, a composition comprises a plurality of microspheres, wherein the microspheres have an actual to predicted surface area ratio of about 3 or less. In some embodiments of the present invention, a composition comprises a plurality of microspheres, wherein the microspheres have an actual to predicted surface area ratio of about 2 or less. The actual to predicted surface area ratio is calculated by dividing the actual surface area as determined by Nitrogen Gas Adsorption divided by the predicted surface area determined by laser particle size analysis. These methods and calculations are set forth herein in Example 2.

In some embodiments of the present invention, a composition comprises a plurality of microspheres, wherein the microspheres are water soluble. Insoluble materials can interfere with the full delivery of an API. Insoluble materials can also become an issue in the formation of complete solutions, plugging needles and filters.

**Microsphere Material**

In some embodiments, the properties of material candidates for use in the present invention is a material that melts at less than 250 °C or a material that dissolves in, melts with or disperses in the primary material. In some embodiments, the material solidifies from a melt on cooling. In some embodiments, the material solidifies from a melt on cooling, in less than 10 minutes. In some embodiments, cooling can be achieved by chilled or non-chilled air, chilled gases or chilled liquids in which the material has limited solubility. In some embodiments, the solidification process forms very thin and parallel deposited crystalline plates that assimilate with each other into a tight crystal packing group. In some embodiments, the material that forms the microsphere is a substance with a trans chemical structure with a molecularly balanced charge distribution and thus a low dielectric constant (< 10). In some embodiments, the material is made up of preferred similar sized and preferred uniformly distributed functional groups, like hydroxyl groups, that do not hinder greatly the bond formation process on cooling, and can form bonding structures rapidly through, as an example, hydrogen bonding almost instantly. In some embodiments, the microsphere material is based on fusion, co-crystallization and/or occlusion of the material into the crystal structure.

In some embodiments, microspheres of the present invention can be comprised of many different materials including, but not limited to, carbohydrates, polyols, sugars, starches, waxes, polyethylene glycol, cetyl alcohol, stearic acid, fatty acids, fatty acid esters, polyethylene glycol derivatives, materials miscible with these materials, or combinations thereof. In some embodiments, the microspheres do not include cellulose. In some embodiments, the microspheres do not include lipids. In some embodiments, microspheres of the present invention can be comprised of one or more materials that melt. In some embodiments, microspheres of the present invention can be comprised of a material that is solid at room temperature. In some embodiments, microspheres of the present invention can be comprised of one or materials that are crystalline solid at room temperature. In some embodiments, microspheres of the present invention can be comprised of one or more amorphous materials, such as melts.

In some embodiments, microspheres of the present invention can also contain additives including, but not limited to, maltodextrins, microcrystalline cellulose, hydroxypropyl methyl cellulose, methyl cellulose, polyvinyl alcohol, sodium CMC, povidone and other vinyl derivatives, calcium carbonate, tartaric acid, alginic acid, talc, titanium oxide, color, flavor, sodium lauryl sulfate, ph adjusters, surface active agents or combinations thereof.

In some embodiments of the present invention, a composition comprises a plurality of microspheres, wherein the microspheres comprise a single core material. In another embodiment, composition comprises a plurality of microspheres, wherein the microspheres comprise a core material, and wherein the core material is a polyol. In another embodiment, a composition comprises a plurality of microspheres, wherein the microspheres comprise a core material, and wherein the core material is mannitol. In another embodiment, a composition comprises a plurality of microspheres, wherein the microspheres comprise a core material, and wherein the core material is 100 % mannitol. In another embodiment, a composition comprises a plurality of microspheres, wherein the microspheres comprise a core material, and wherein the core material is sorbitol. In another embodiment, a composition comprises a plurality of microspheres, wherein the microspheres comprise a core material, and wherein the core material is maltitol. In another embodiment, a composition comprises a plurality of microspheres, wherein the microspheres comprise a core material, and wherein the core material is isomalt. In another embodiment, a composition comprises a plurality of microspheres, wherein the microspheres comprise a core material, and wherein the core material is erythritol. In another embodiment, a composition comprises a plurality of microspheres, wherein the microspheres comprise a core material, and wherein the core material is xylitol. In another embodiment, a composition comprises a plurality of microspheres, wherein the microspheres comprise one or more core materials. In some embodiments of the present invention, a composition comprises a plurality of microspheres, wherein the microspheres have a crystalline structure.

In some embodiments of the present invention, a composition comprises a plurality of microspheres, wherein the microspheres comprise a single material. In another embodiment, a composition comprises a plurality of microspheres, wherein the microspheres comprise a polyol. In another embodiment, a composition comprises a plurality of microspheres, wherein the microspheres comprise mannitol. In another embodiment, a composition comprises a plurality of microspheres, wherein the microspheres comprise 100 % mannitol. In another embodiment, a composition comprises a plurality of microspheres, wherein the microspheres comprise sorbitol. In another embodiment, a composition comprises a plurality of microspheres, wherein the microspheres comprise maltitol. In another embodiment, a composition comprises a plurality of microspheres, wherein the microspheres comprise erythritol. In another embodiment, a composition comprises a plurality of microspheres, wherein the microspheres comprise isomalt. In another embodiment, a composition comprises a plurality of microspheres, wherein the microspheres comprise xylitol.

In another embodiment, a composition comprises a plurality of microspheres, wherein the microspheres comprise one or more polyols. In another embodiment, a composition comprises a plurality of microspheres, wherein the microspheres comprise mannitol and sorbitol. The ratio of mannitol to sorbitol can vary. In one embodiment, a composition comprises a plurality of microspheres, wherein the microspheres comprise mannitol and sorbitol, wherein the mannitol:sorbitol ratio ranges from about 99.5:0.5 to about 90:10. In another embodiment, a composition comprises a plurality of microspheres, wherein the microspheres comprise mannitol and xylitol. The ratio of mannitol to xylitol can vary. In one embodiment, a composition comprises a plurality of microspheres, wherein the microspheres comprise mannitol and xylitol, wherein the mannitol:xyltiol ratio ranges from about 99.5:0.5 to about 90:10. In another embodiment, a composition comprises a plurality of microspheres, wherein the microspheres comprise mannitol and erythritol. The ratio of mannitol to erythritol can vary. In one embodiment, a composition comprises a plurality of microspheres, wherein the microspheres comprise mannitol and erythritol, wherein the mannitol:erythritol ratio ranges from about 99.5:0.5 to about 90:10. In another embodiment, a composition comprises a plurality of microspheres, wherein the microspheres comprise mannitol and maltitol. The ratio of mannitol to maltitol can vary. In one embodiment, a composition comprises a plurality of microspheres, wherein the microspheres comprise mannitol and maltitol, wherein the mannitol:maltitol ratio ranges from about 99.5:0.5 to about 90:10. In another embodiment, a composition comprises a plurality of microspheres, wherein the microspheres comprise mannitol and isomalt. The ratio of mannitol to isomalt can vary. In one embodiment, a composition comprises a plurality of microspheres, wherein the microspheres comprise mannitol and isomalt, wherein the mannitol:isomalt ratio ranges from about 99.5:0.5 to about 90:10. In some embodiments, a composition comprises a plurality of microspheres, wherein the microspheres consist of a polyol. In some embodiments, a composition comprises a plurality of microspheres, wherein the microspheres consist of two or more polyols. In some embodiments, a composition comprises a plurality of microspheres, wherein the microspheres consist of mannitol. In some embodiments, a composition comprises a plurality of microspheres, wherein the microspheres consist of a mannitol and sorbitol. In some embodiments, a composition comprises a plurality of microspheres, wherein the microspheres consist of maltitol. In some embodiments, a composition comprises a plurality of microspheres, wherein the microspheres consist of erythritol. In some embodiments, a composition comprises a plurality of microspheres, wherein the microspheres consist of xylitol. In some embodiments, a composition comprises a plurality of microspheres, wherein the microspheres consist of isomalt. In some embodiments, a composition comprises a plurality of microspheres, wherein the microspheres consist of mannitol and xylitol. In some embodiments, a composition comprises a plurality of microspheres, wherein the microspheres consist of mannitol and erythritol. In some embodiments, a composition comprises a plurality of microspheres, wherein the microspheres consist of mannitol and maltitol. In some embodiments, a composition comprises a plurality of microspheres, wherein the microspheres consist of mannitol and isomalt.

**Methods of Manufacture**

In some embodiments, microspheres of the present invention can be manufactured by various methods. In one embodiment, microspheres can be produced by prilling (spray chilling) of a melt of core material. In this embodiment, a melted material is poured into a heated pressure vessel. The heated pressure vessel is pressurized and the plug valve at the bottom of the vessel is opened to send the melt thru the spray line to the nozzle. The spray line is heated if necessary. The nozzle can also be heated if needed. The prills from the nozzle are collected.

In another embodiment, microspheres of the present invention can be made by melting a material and dropping the melt onto a spinning spin disc for formation of microspheres. The material is melted. In some embodiments, the material can be melted via pan, oven or use of a powder extruder to melt system, such as is available from Randcastle extruders (RCP-1000) (Cedar Grove, NJ) to decrease time of material at melt.

In this embodiment, powder is fed in and RPM of unit is set to control feed rate of melt. Residence time of melt can be < 2 minutes in unit and flow rate is consistent.

In another embodiment, microspheres can be made by melting a material as a powder in the spin disk head assembly (See Gold Metal Cincinnati Ohio Tornado unit). Once melted, the liquid material is spun into a stream which, by centrifugal force, is spread into a thin film and exits the disk as a ligament that breaks into droplets or exits as droplets. In a preferred embodiment, a surface spinner style disk is preferred with a diameter of 4 inches or more and speed capabilities of from 200 RPM to 11,000 RPM. The wheel RPM controls film thickness and thus droplet/bead/microsphere size. The microspheres are allowed to fall in room temperature or chilled air to cool. In some embodiments, once cooled, any twinning or chill damaged beads if present, can be removed with a bead shape shorter.

In another embodiment, microspheres of the present invention can be made by using a sonic nozzle system supplied by Brace (Alzenan, Germany) called the Brace Spherisator M. The unit consists of an oven in which one or two liquid vessels are stored. The oven can be heated up to 200°C. Thus thermal conditions can be maintained on the liquid to 200°C. In some embodiments, the bottle(s) containing the melt can be pressurized. If the head space is pressurized, the liquid will flow to a nozzle which is mounted in a sonicator. Both the amplitude and frequency of the sonic energy can be adjusted. The motion as the liquid exits the nozzle causes the liquid stream to separate and form drops. In some embodiments, a strobe can be used to see the droplets form. Based on amplitude as a gross adjustment and frequency as the finer adjustment, the droplets release as individuals approximately the size the droplet would be as a cylinder and surface tension then coverts it to a sphere.

In some embodiments, mannitol alone in a single nozzle or an API dissolved in or dispersed in the mannitol melt made into microspheres using this approach produce microspheres either as a pure mannitol microsphere or as a mannitol and API dispersion. In some embodiments, phenytoin, carbamazepine and folic acid are examples of APIs that can be dissolved/dispersed in the melt of mannitol at 180°C. The liquid is delivered from a single sonic nozzle. The nozzle vibrates in an up and down amplitude at a frequency to produce an individual droplet at the tip. The droplet is allowed to cool as it freely falls to form a solidified microsphere. Cooling can occur at room temperature or in a chilled environment. In some embodiments, as a signal nozzle setup using a 200 $\mu$m nozzle at a flow rate of 35 g/min with the amplitude and frequency set based on a strobe light to maintain droplet formation separation. Pressure on the vessel is maintained to maintain flow rate.

In some embodiments, a liquid can also be injected in a center nozzle of a two concentric nozzle setup. In one embodiment, the 100 $\mu$m outer and the 100 $\mu$m inner nozzle in the Spherisator M. The material can be a melted API or a non-volatile liquid containing dissolved API or a nanosized API suspension or mannitol melt or mannitol melt API solution or dispersion. The material is delivered under pressure from a vessel in an oven to maintain its temperature needed to maintain the melt. A coating of a material melt alone or coating of an API dissolved or dispersed in the material melt can form the outer shell.

**Applications**

The microspheres of the present invention are useful in various applications. In one embodiment, the microspheres of the present invention are useful in the manufacture of sustained and modified release beads for dosing active pharmaceutical ingredients (APIs) as multiparticulate systems. In another embodiment, the microspheres of the present invention are useful are carriers for APIs for subsequent manufacture into tablets. In another embodiment, the microspheres of the present invention are useful as a free flowing excipient in the manufacture of mini-tablets. In another embodiment, the microspheres of the present invention are useful in the manufacture of API dispersions.

In some embodiments, microspheres of the present invention are useful as core beads onto which an API is layered either in a suspension or a solution or dry powder alternated with a solution to create a tacky surface and if needed a functional coating also applied. In some embodiments, microspheres of the present invention are useful as core beads for immediate, modified and/or sustain release active and coated beads for inclusion into sachets, capsules and tablet formulations. In some embodiments, microspheres of the present invention are useful as placebo beads. In some embodiments, microspheres of the present invention are useful as cores for plating of APIs onto by lyophilization process.

In some embodiments of the present invention, microspheres can have a small particle size. In some embodiments, such microspheres of the present invention are useful for sachets and chewable tablets to reduce damage [to the API] during chewing, and to improve the mouthfeel of the tablet. In some embodiments, such microspheres are useful in all dosage forms to reduce final bead size yet allow for high API dose loading. In some embodiments, such microspheres are useful as they may allow for a greater thickness of API coating and thus a wider range of release rate options for use of thicker coatings giving a slower release. In some embodiments, such small microspheres are useful as they may reduce localized concentration of irritative drug by providing greater surface area. In some embodiments, small microspheres are useful as they may reduce variation in gastric emptying rate and transit time. In some embodiments, small microspheres are useful as they are less susceptible to dose dumping. In some embodiments, small microspheres are useful as they disperse more freely in gastrointestinal tract and invariably maximize API absorption and also reduce peak plasma fluctuation. In some embodiments, small microspheres are useful as they can be used as a free flowing excipient in mini-tablets.

## Compositions

In some embodiments of the present invention, pharmaceutical compositions comprise microspheres of the present invention and an active pharmaceutical ingredient (API). In some embodiments, pharmaceutical compositions comprise a plurality of microspheres and API. APIs useful in the present invention may include but are not limited to those described in the Physician's Desk Reference, 61st ed. Montvale, NJ: Thomson PDR; 2007, which is incorporated by reference herein in its entirety. In some embodiments, the API may be present inside the microsphere. In another embodiment, the API may be present on the outside of the microsphere. In some embodiments, the microspheres of the present invention are useful in combination with standard methods of API incorporation into or onto beads or microspheres.

In some embodiments of the present invention, a blend of powder of a core material and an API can be added to a melt extruder and once melted would disperse in melt and discharge as a melt stream either to be pressure atomized or to a spin disc for creation of microspheres. In another embodiment, an API can be dissolved in a melted core material. In another embodiment, a second feeder position can be added to a two-stage melt extruder wherein the melt flowing at a controlled rate based on RPM of the melt extruder push melt past a powder entry point where a mixture of the API and as an option additional core material or other additives are delivered. The melt and API or API dispersion is then transferred though a mixing section of the extruder and then delivered to either the spin disc or to a pressurized atomizer unit.

## Conventional Pan System

In some embodiments, microspheres can be coated using a conventional pan system. The standard coating pan system consists of a circular metal pan mounted sat an angle on a stand, the pan is rotated on its horizontal axis by a motor, the hot air is directed into the pan and onto the bed surface, and is exhausted by means of ducts positioned through the front of the pan. Coating solutions are applied by spraying the material on the bed surface. As coating is applied the coating solution is dried off. It is common to dust powder mixtures onto wetted beads and dry the beads in layers. A final color and seal coating is often applied.

## The Perforated Coating Pan

In some embodiments, microspheres can be coated using a perforated coating pan. Coating pan has perforations along its cylindrical portion. It is driven by a variable speed drive. Supply of hot air and exhaust of drying air are arranged to facilitate the coating system through stainless steel plenums positioned on both sides of the perforated coating pan. The pan is enclosed in an airtight housing provided with a suitable door and front glass window. This housing of pan with drive is a stainless steel cabinet accommodating the gearbox, AC variable drive, power panel, hot air unit, exhaust unit and an air fitter.

Liquid spray system is complete with stainless steel liquid storage vessel, variable flowrate liquid dosing pump, automatic spray gun, and inter-connecting flexible hoses.

## The Fluidized Bed Coater

In some embodiments, the Fluid Bed Technology can be used to coat microspheres. The Fluid Bed Technology is the more modern approach to coating beads. It is a very efficient coating technique. The major advantage of the Fluid Bed Systems it is a closed system that air suspends the beads.

In a fluidized bed a coat is introduced to cover the core particles inside the bed. In the process, a layer is deposited onto the surface of fluidized solid particles by spraying with a solution of the coating material. The fluidizing gas is also use to dry the deposited solution. There is considerable diversity in methods of using fluidized bed technology. For e.g. liquids can be applied to fluidized particles in a variety of ways, including top, bottom and tangential spraying.

For a given product, each method can offer markedly different finished product characteristics.

Fluidized beds are used for coating because of their high energy and mass transfer. Fluidized beds for film coating can be divided into three groups: top spray, tangential spray, and bottom-spray equipment.

In the top spray bed, the expansion chamber is lengthened to allow powder to remain fluidized longer and to move with a higher velocity, so that agglomeration is minimized. The expansion chamber is conically shaped to allow uniform deceleration of air stream. The filter housing is larger and designed to shake the fines back into the bed interrupting fluidization; this reduces agglomeration tendencies. The nozzle is positioned low in the expansion chamber so that coating material impinge on the fluidized particle a short distance from the nozzle; this reduces droplet spray drying and provides for longer subsequent drying of the coated particles. The top spray coater has been used to apply aqueous and organic solvent based film coatings, controlled release coatings. Smaller microspheres in this technique would allow smaller final beads and/or thicker coatings.

In the bottom spray coating, the Wurster machine employs a cylindrical product container with a perforated plate. Inside the container is a second cylinder (coating partition) with is raised slightly above the perforated plate, centered in the plate below this partition is a spray nozzle used to dispense the coating solution. The perforated plated is designed with large holes in the area under the coating partition and smaller holes in the remainder of the plate, except for one ring of large holes at the perimeter. The design allows the core particles to be pneumatically transported upward through the coating partition, and downward outside this partition. Material passing through coating partition receives a layer of coating material, dries in the expansion chamber, and falls back in a semi fluidized state. Material circulates rapidly in this fashion and receives layer of coating material, dries in the expansion chamber, and falls back in a semi fluidized state material circulates rapidly in this fashion and receives a layer of coating on each pass through the coating partition. The ring of large holes on the periphery of perforated plate prevents the accumulation of material at the container wall. It is used for coating small particles, beads, tablets and capsules.

The tangential spraying system, which is commonly fitted with a rotating bottom plate, can achieve film quantities nearly as good as the bottom spraying system. The rotation of the plate nicely supports product movement, so that the required air amount is mainly used for drying process and only to a smaller degree for the product movement.

**Fluid Bed Coating**

In some embodiments, microspheres can be coated using a fluid bed coating system. In some embodiments, for microspheres of small sizes the coating film is used to control the release rate of the API. Microspheres allow the loading of the API in a coating layer first up to 1 to 2 mm in diameter followed by the application of the release controlling layer. Thus the small particle diameter of the microsphere add to API loading capacity and the decreasing particle diameter and thus the specific surface area of a substrate increase dramatically and the required coating weight gain is not experienced. Drug layering can be applied more rapidly than the final controlled coating layer.

**Rotating Disk Granulation**

In some embodiments, microspheres can be coated using granulation. Granulation techniques utilizing centrifugal fluidizing disk that can be moved up or down to create a variable slit opening between the outer perimeter of the disk and the sidewall of the container. Air is drawn into the product container through the slit under negative pressure. This fluidizes the material along the circumferential surface of the product container. At the same time the disk rotates at varying speeds and moves the product by the centrifugal force to the outer portions where it is lifted by the fluidizing air stream into the expansion chamber. As the material decelerates, it descends to the center of the disk and repeats the same sequence.

The fluidization pattern is often described as a spiraling helix or rope-like pattern around the inside of the rotor chamber.

Spray nozzles can be immersed in the bed of fluidized material and spray applied in tangential fashion with respect to the particle flow. Microspheres in this process allows for a starting controlled surface onto which the coating powder with API can be layered onto and held by a spray solution of coating materials in a rapid layering application approach. Based on the uniformity of both shape and size these microspheres allow for a uniform and rapid gain in weight and maintain separation readily versus standard crystal seeds currently used and starter material.

**Dosage Forms**

The various embodiments of the composition, according to the present invention, may be used in a variety of dosage forms including, but not limited to, chewable tablets, swallow tablets, soft chews including tablets and soft gel capsules, orally disintegrating tablets, orally dispersible powders, mini-tablets, lozenges, film strips, gums, gels, ointments and creams, tablet inserts (eye, ear, vaginal), suppositories, hard shell capsules, liquid fill capsules, liquid suspensions and sustained release beads.

In some embodiments, the dosage form may include a pharmaceutically acceptable ingredient including excipients, orally disintegrating excipients, including, but not limited to Pharmaburst 500 (SPI Pharma, Inc.); diluents; disintegrants; binders; fillers; bulking agent; organic acid(s); colorants; stabilizers; preservatives; lubricants; glidants/anti-adherants; chelating agents; vehicles; bulking agents; stabilizers; preservatives; tonicity adjusting agents; local anesthetics; pH adjusting agents; antioxidants; osmotic agents; chelating agents; viscosifying agents; wetting agents; emulsifying agents; acids; sugar alcohol; reducing sugars; non-reducing sugars and the like used either alone or in combination thereof. In some embodiments, the pharmaceutically acceptable ingredients may include excipients, binders, lubricants, sugar alcohols, disintegrating agents, colors, flavors and the like used either alone or combinations thereof.

In some embodiments, a pharmaceutical formulation comprising a plurality of microspheres may be used in a directly compressible dosage form. The term "directly compressible" means that the composition can be compressed to tablet form on standard tableting machines (including, but not limited to high speed tableting machines) using standard (i.e., without any specially machined, shaped or coated surfaces) punches and dies, without any significant amount of the composition adhering to the punches and dies by applying compressive pressure to the composition. In some embodiments, the compression pressure ranges from 60 MPa to 170 MP. In some embodiments, the compression force ranges from 80 MPa to 150 MPa. In some embodiments, the compression pressure is at least 60 MPa.

The term "pharmaceutical formulation" as used herein refers to formulations containing the composition of the present invention in combination with carriers or excipients suited to a selected drug delivery platform, e.g., a capsule, an orally dispersible formulation, an effervescent formulation, a chewable tablet, a lozenge, a hard or swallow tablet, or the like.

"Carriers" or "vehicles" as used herein refer to carrier materials suitable for oral drug administration, and include any such materials known in the art, e.g., diluents, binders, granulating agents, disintegrants, lubricating agents, colorants, flavoring agents, and the like.

In some embodiments, various types of pharmaceutical formulations may be prepared using the presently disclosed microspheres and compositions, including powders, chewable tablets, orally dissolving tablets, effervescent formulations, and liquid dispersions. For solid formulations such as powders, chewable tablets, orally dissolving tablets and effervescent formulations, conventional carriers, excipients and additives can be employed, including diluents, binders, granulating agents, disintegrants, flavoring additives, and the like. Examples of the normally employed excipients include pharmaceutical grades of mannitol, lactose, starch, and the like. Liquid pharmaceutical compositions containing the present microspheres will generally be prepared by dispersing or suspending the microcapsules in a non-aqueous carrier which does not cause release of the drug, or else by dispersing the microspheres or composition in an aqueous carrier immediately prior to administration to the patient. In some embodiments, the microspheres or composition may be provided as a free-flowing particulate material, as in a sachet or other suitable package, and such a particulate material may be dispersed in an aqueous carrier. These solid or liquid formulations may contain any amount of the microsphere or composition needed to provide the desired amount of the active ingredient contained in the microsphere or composition. In some embodiments, amounts of microspheres or composition on the order of about 10 wt. % to about 95 wt. % of the dosage form may be used. Actual methods of preparing such dosage forms are known, or will be apparent, to those skilled in this art.

It will be apparent to one of skill in the art that various modifications and variations can be made in the methods and compositions of the present invention without departing from the spirit or scope of the invention. Thus, it is intended that the present invention cover the modifications and variations of the present invention provided they come within the scope of the appended claims and their equivalents.

**EXAMPLES**

The following examples are given to illustrate the present invention. It should be understood, however, that the invention is not to be limited to the specific conditions or details described in these examples. Throughout the specification, any and all referenced publicly available documents, including but not limited to a U.S. patent, are specifically incorporated by reference.

Example 1

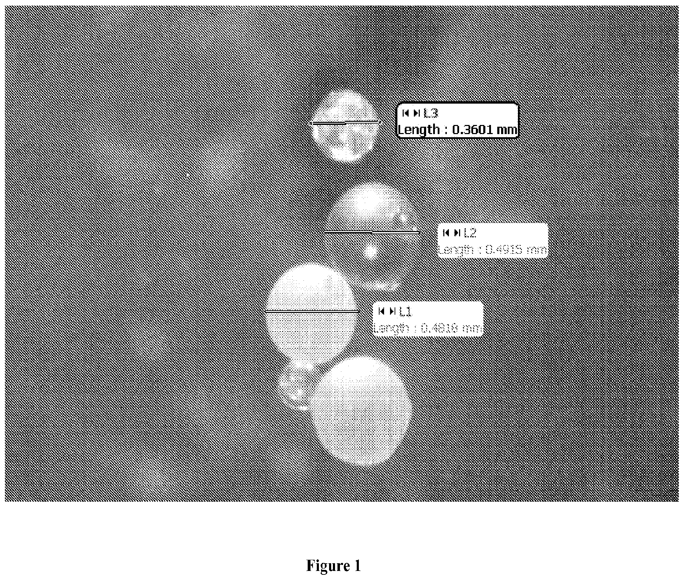

Mannitol (EP) (Shandong Tianli Pharmaceutical Ltd., Guangzhou, China) and mannitol/sorbitol (2.3%) (sorbitol, Roquette, Keokuk , Iowa) microspheres were produced by prilling (spray chilling) of a melt of the polyols in a stainless steel 2 quart sauce pan. The melted polyol was poured into a heated pressure vessel that was heated by electrical heat bands. The heated pressure vessel was pressurized to 50 psig and the plug valve at the bottom of the vessel was opened to send the mannitol thru the spray line to the nozzle. The spray line was heated by electrical heat tape. The nozzle was heated with a propane torch prior to opening the valve. The prills from the nozzle (Spraying Systems 6501) were collected on plastic and bagged for evaluation. Microspheres particle size distribution was determined by Malvern Mastersizer Laser analysis (Malvern, PA). Table 1 shows the particle size distribution of the microspheres. Microsphere diameters were in the 250 $\mu$m mean range with a broad distribution (d(0.1) = 124 and d(0.9) = 473 um or 473/124 = 3.8 to 1 distribution ratio and d(0.1) = 204 and d(0.9) = 598 um or 598/204 = 2.9 to 1 distribution ratio. Figure 1 shows a photomicrograph of the microspheres demonstrating a total smooth glass-like surface of the microspheres. (Carl Zeiss Microscope Model Axio Vert.A1 (Oberkochen, Germany)).

Table 1: Particle size distribution

| Sample | d(0.1) | d(0.5) | D(0.9) | PSD Ratio |
|---|---|---|---|---|
| Sample 1 (Mannitol) | 124 | 250.6 | 473 | 3.8 |
| Sample 2 (Mannitol) | 122 | 261.6 | 494 | 4.0 |
| Sample 3 (Mannitol/Sorbitol) | 164 | 323 | 567 | 3.45 |
| Sample 4 (Mannitol/Sorbitol) | 96 | 225 | 451 | 4.7 |

Example 2

Mannitol EP (Shandong Tianli Pharmaceutical Ltd., Guangzhou, China), mannitol/sorbitol (2.3%) (sorbitol, Roquette, Keokuk, Iowa) and mannitol/polysorbate 80 (Unichema, New Castle, DE) microspheres were made by melting polyol and dropping melt to spin disc for formation of microspheres. Mannitol is melted in pan or oven at 10°C above its melting point for mannitol a temperature higher than 176°C. Once melted, the liquid mannitol is spun into a stream which, by centrifugal force, is spread into a thin film and exits the disk as a ligament that breaks into droplets or exits as droplets. Surface spinner style disk is preferred with a diameter of 4 inches or more and speed capabilities of from 500 RPM to 11,000 RPM. Wheel RPM controls film thickness and thus droplet/bead size. Microspheres are allowed to fall at least 8 feet in room temperature or chilled air to congeal. Once congealed a coarse screen can be used to complete cooling and maintain separation. Any twinning or chill damaged microspheres are removed with a bead shape shorter.

Microspheres were analyzed for particle size using Malvern Mastersizer. Table 2 shows the particle size distribution (PSD) of the microspheres.

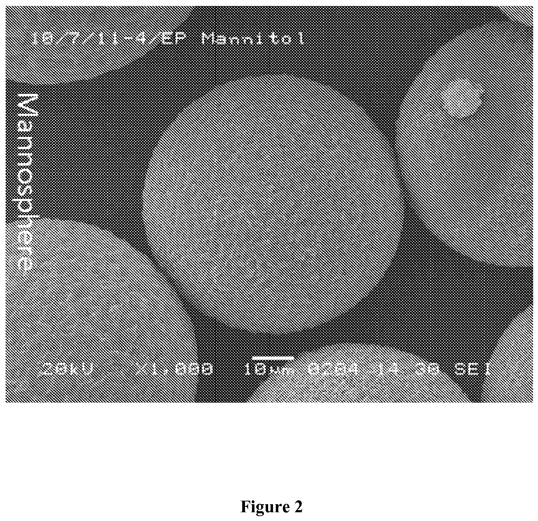

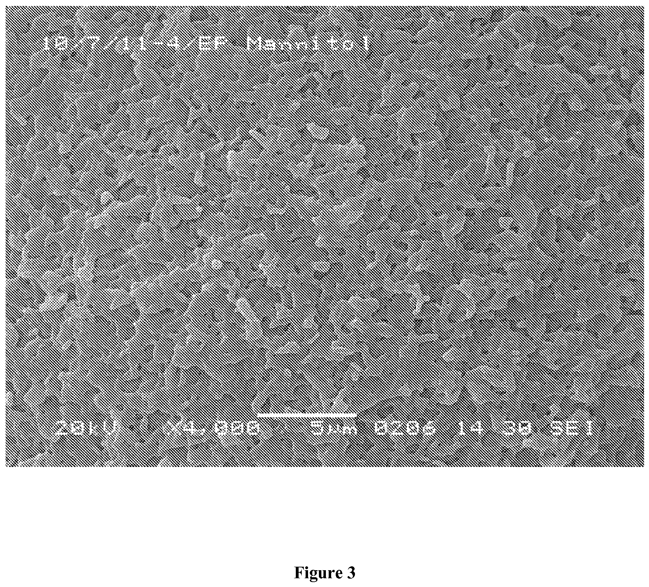

Scanning electron microscopy (SEM) was performed on exemplary mannitol microspheres. Figure 2 is a close up (1,000x magnification) micrograph of SEM (with 10$\mu$m measurement bar) of the mannitol microspheres made by this process. Note the surface flat, crystal plates with ridges present, the perfect circularity and sphericity and the lack of crevices and risers. The frequency of the ridges seen in Figure 2 are greater than about one per $\mu$m of distance along a surface of the microsphere (i.e., > about 10 per the length of the 10$\mu$m bar as shown in Fig 2 along a surface of the microsphere). Figure 2 shows that all sizes of particles have similar ridge texturing. Figure 3 is a close up (4,000x magnification) micrograph of SEM (with 5 $\mu$m measurement bar) of the surface of the center mannitol microsphere shown in Figure 2. Note the flat crystal plates that form ridges on the surface of the microsphere. The flat crystal plates orient in a horizontal direction either during the droplet formation (due to spinning orientation) and/or cooling (surface nucleation orientation) of the droplet to form the solid microsphere. The ridges are less than 1 $\mu$m in height. The frequency of the ridges as seen in Figure 3 are greater than about one per $\mu$m of distance along a surface of the microsphere (i.e., > about 5 per the length of the 5 $\mu$m bar as shown in Fig. 3 along a surface of the microsphere).

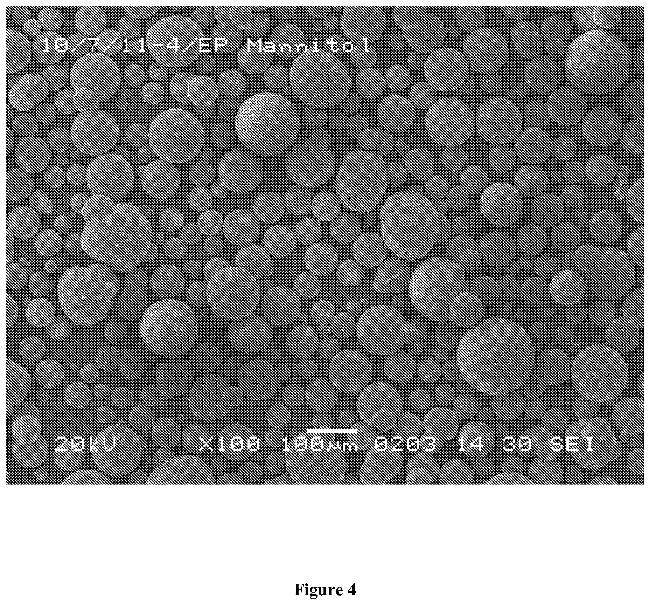

Figure 4 is a micrograph of SEM, which shows the size distribution of mannitol microspheres available with this process and the ability to achieve microspheres at sizes down to about 10 $\mu$m and smaller. Note in this figure, the uniformity of shape and circularity/sphericalness even at and down to the 10 $\mu$m level. Figure 4 also shows that all sizes of the microspheres contain a similar very shallow but rough surface based on these flat crystal plate ridges being present and none, in this embodiment, are glass surface smooth.

Table 2: Particle size distribution

| Sample | d(0.1) | d(0.5) | D(0.9) | PSD Ratio |
|---|---|---|---|---|
| | 133.6 | 211.7 | 297.2 | 2.2 |
| Run 1 -Mannitol EP | Disc Speed: 2000 RPM | | Flow Rate: 110 gm/min | |
| | 120 | 223 | 395 | 3.3 |
| Run 2-Mannitol EP | Disc Speed: 1500 RPM | | Flow Rate: 110 gm/min | |
| | 126 | 216 | 354 | 2.8 |

(continued)

| Sample | d(0.1) | d(0.5) | D(0.9) | PSD Ratio |
|---|---|---|---|---|
| Run 3-Mannitol EP | Disc Speed: 1250 RPM | | Flow Rate: 110 gm/min | |
| | 162 | 273 | 456 | 2.8 |
| Run 4-Mannitol EP | Disc Speed: 1000 RPM | | Flow Rate: 110 gm/min | |
| | 226 | 407 | 672 | 2.97 |
| Run 5-Mannitol EP | Disc Speed: 750 RPM | | Flow Rate: 110 gm/min | |
| | 299 | 499 | 777 | 2.6 |
| Run 6-Mannitol EP | Disc Speed: 2000 RPM | | Flow Rate: 49 gm/min | |
| | | | | |
| Run 7-Mannitol EP | Disc Speed: 1500 RPM | | Flow Rate: 49 gm/min | |
| | 178 | 303 | 494 | 2.8 |
| Run 8-Mannitol EP | Disc Speed: 1250 RPM | | Flow Rate: 49 gm/min | |
| | 221 | 373 | 602 | 2.7 |
| Run 9-Mannitol EP | Disc Speed: 1500 RPM | | Flow Rate: 110 gm/min | |
| | 146 | 299 | 531 | 3.6 |
| Run 10-Mannitol EP | Disc Speed: 1500 RPM | | Flow Rate: 49 gm/min | |
| | 178 | 303 | 494 | 2.8 |
| Run 11 -Mannitol EP | Disc Speed: 1500 RPM | | Flow Rate: 110 gm/min | |
| | 248 | 447 | 730 | 2.9 |
| Run 12-Mannitol EP | Disc Speed: 1500 RPM | | Flow Rate: 49 gm/min | |
| | 162 | 277 | 469 | 3.1 |
| Run 13-Mannitol EP | Disc Speed: 1500 RPM | | Flow Rate: 200 gm/min | |
| | 127 | 252 | 478 | 3.8 |
| Run 14-Mannitol EP | Disc Speed: 1500 RPM | | Flow Rate: 163 gm/min | |
| | 150 | 310 | 564 | 3.76 |
| Run 1-Mannitol EP (10/7) | Disc Speed: 11000 RPM | | Flow Rate: 110 gm/min | |
| | 29 | 57 | 105 | 3.6 |
| Run 2-Mannitol EP (10/7) | Disc Speed: 11000 RPM | | Flow Rate: 163 gm/min | |
| | 27 | 58 | 117 | 4.3 |
| Run 3-Mannitol EP (10/7) | Disc Speed: 11000 RPM | | Flow Rate: 200 gm/min | |
| | 29 | 63 | 123 | 4.24 |
| Run 4-Mannitol EP (10/7) | Disc Speed: 5000 RPM | | Flow Rate: 200 gm/min | |
| | 33 | 66 | 129 | 3.9 |
| Run 5-Mannitol/Sorbitol | Disc Speed: 5000 RPM | | Flow Rate: 200 gm/min | |
| | 35 | 74 | 138 | 3.9 |
| Run 6-Mannitol/ Sorbitol | Disc Speed: 11000 RPM | | Flow Rate: 300 gm/min | |
| | 24 | 62 | 130 | 5.4 |
| Run 7-Mannitol w/ polysorbate 80 | Disc Speed: 5000 RPM | | Flow Rate: 200 gm/min | |

(continued)

| Sample | d(0.1) | d(0.5) | D(0.9) | PSD Ratio |
|---|---|---|---|---|
|  | 35 | 74 | 138 | 2.5 |

**Karl Fisher Moisture (USP)**

Approximately 1.0 g of the mannitol EP microspheres, Colorcon SureSpheres® sugar/starch spheres (Colorcon, West Point, PA), and Werner Pharm-a-Sphere™ Neutral Pellets (Hanns G. Werner GmbH, Tornesch, Germany) were analyzed for moisture content using in the Karl Fisher method described in US Pharmacopeia (USP) 26. Table 3 shows the moisture content of the mannitol microspheres, SureSpheres, and Pharm-a-Spheres.

Table 3: Moisture content of mannitol microsphere vs. commercial beads

| Product | Mass (g) | Titrant (ml) | Water (%) |
|---|---|---|---|
| SureSphere | 1.0053 | 2.560 | 1.32 |
| Pharm-a-Sphere Neutral Pellets | 1.0032 | 1.578 | 0.81 |
| Mannitol (USP) Microspheres (10/7/1-6) | 0.9988 | 0.184 | 0.1 |
| Mannitol (EP) Microspheres (10-6-9) | 1.0087 | 0.036 | 0.02 |

**Dynamic Vapor Sorption**

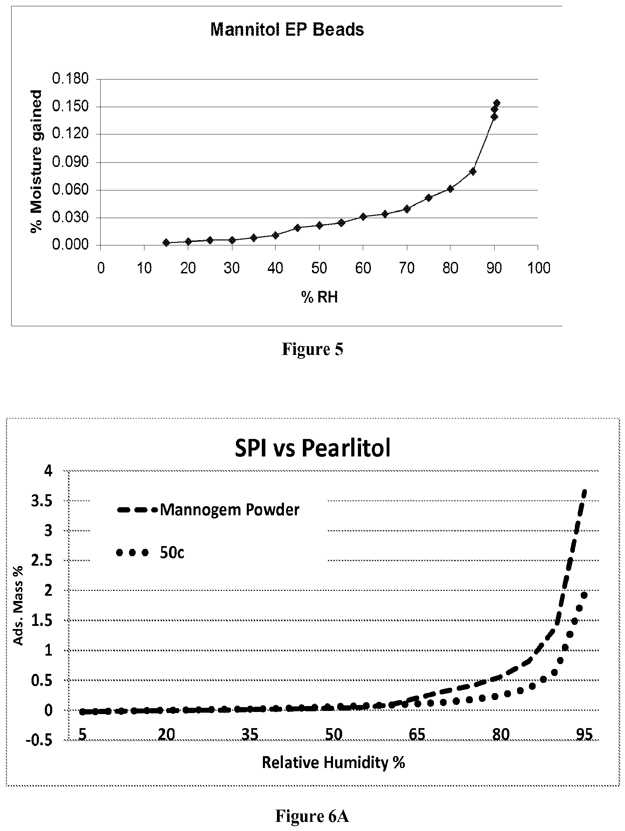

0.2 g of the mannitol EP microsphere was analyzed on an AquaDyne Instrument of the Quantachrome (Quantachrome Instruments Palm Beach Florida) using the aquaWin - Data Acquisition and Reduction to measure dynamic vapor sorption. Figure 5 shows the hygroscopicity of mannitol microspheres. Results show that mannitol microspheres are extremely non-hygroscopic at normal process conditions of 60% relative humidity (RH) as weight gain is less than 0.05%. Results also show a lack of moisture adsorbing pore sizes as beads even at 90% RH gained less than 0.2% moisture.

In order to compare the hygroscopicity of the mannitol microspheres to other forms of mannitol, powdered (Mannogem® powder, Lot # 121101399F, SPI Pharma, Inc.; Pearlitol 50C, Lot # KVKRN, Roquette Freres), granular (Mannogem granular, Lot # 121101116G, SPI Pharma, Inc.; Pearlitol 400DC, Lot # E592J, Roquette Freres), and sprayed-dried mannitol (Mannogem EZ, Lot # 121101324, SPI Pharma, Inc.; Pearlitol 200 SD, Lot # E983G, Roquette Freres) products were submitted to Quantachrome for Dynamic Vapor Sorption (DVS) analysis.

The adsorption profiles for Mannogem powder and Pearlitol 50c are compared in Figure 6A. The profile for Mannogem powder shows that it begins to adsorb more moisture at around 62% relative humidity. In addition, the Mannogem powder reaches a higher adsorption at 95% RH.

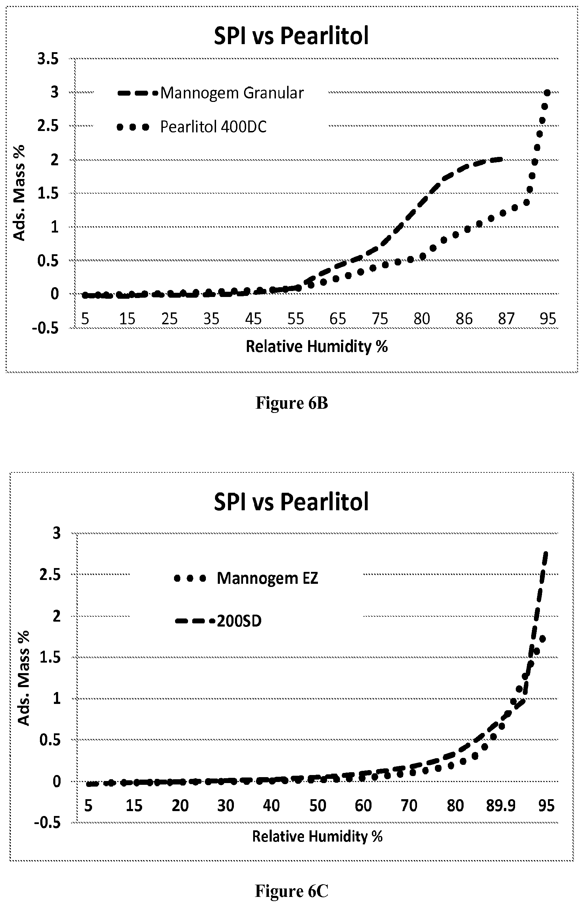

The adsorption profiles for Mannogem granular and Pearlitol 400DC are compared in Figure 6B. Similar to the Mannogem powder profile, the Mannogem granular shows a higher moisture adsorption at around 55% relative humidity. The Mannogem granular appears to stop adsorbing moisture at around 86% RH.

The adsorption profiles for Mannogem spray dried and Pearlitol 200SD are compared in Figure 6C. The profile for Mannogem spray dried shows that the SPI product has a much lower adsorption than the Pearlitol 200SD. The Pearlitol increases in adsorption at around 60% relative humidity. In addition, the Pearlitol 200SD has a much higher adsorption at the highest RH values.

None of the above adsorption profiles for the powder, granular and spray-dried mannitol-based commercial products are below 0.2% moisture content at 95% RH. As seen in Figure 4, the mannitol microspheres of the present invention have a less than 0.2% moisture weight gain at 95% RH compared to over 1.5% for the Mannogem EZ which was the best performer amongst powder, granular and spray dried products tested.

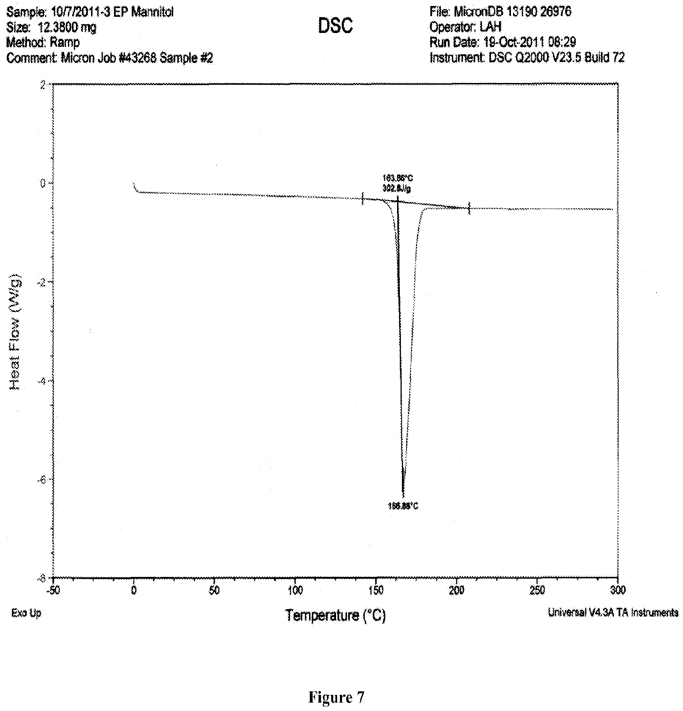

Mannitol microspheres was measured by differential scanning calorimetry (DSC) using a Thermal Analysis (New Castel, DE) DSC Q2000 instrument with a version V23.5 data acquisition system at 10°C per minute from room temperature to 300°C. Figure 7 shows DSC scan of mannitol microsphere at alpha mannitol melt point of 166°C and with heat of fusion at 302 J/gm. From Burger*, alpha mannitol heat of fusion is 285.3 J/gm. Thus bond energy/gm is equal to or more than alpha mannitol. Beta is 293 J/gm as reported by Burger*. This demonstrates that the bond energy in the crystal lattice is equal to or less than that of the microsphere and thus the bead is 100% crystalline with limited to no amorphous regions.

**Skeletal Density**

Mannitol EP microspheres and Nonpareil-108 beads (Freund Industrial Co., Japan) were analyzed for skeletal density using an Ultrapyc 1200e V4.02 of Quantachrome Corporation (Palm Beach, FL). Table 4 shows the skeletal density of mannitol microspheres. Table 5 shows the skeletal density of Nonpareil-108 beads.

Table 4: Skeletal density of mannitol microspheres by helium pycnometry

| Run | Volume (cc) | Density (g/cc) |
|---|---|---|
| 1 | 0.8655 | 1.4651 |
| 2 | 0.8674 | 1.4620 |
| 3 | 0.8674 | 1.4620 |
| 4 | 0.8680 | 1.4609 |
| 5 | 0.8684 | 1.4602 |
| 6 | 0.8692 | 1.4590 |
| 7 | 0.8688 | 1.4595 |
| Average Density: 1.461 g/cc | | |

Table 5: Skeletal density of Nonpareil-108 beads by helium pycnometry

| Run | Volume (cc) | Density (g/cc) |
|---|---|---|
| 1 | 0.3947 | 1.4547 |
| 2 | 0.3947 | 1.4549 |
| 3 | 0.3946 | 1.4552 |
| 4 | 0.3946 | 1.4552 |
| 5 | 0.3945 | 1.4554 |

The closeness of the helium pycnometry skeletal density to the true density of alpha mannitol at 1.468 $gm/cm^{-3}$ (Burger et al.*) versus 1.4590 to 1.4651 for samples indicates microspheres are solid structures and substantially lack interior voids/porosity. Whereas the skeletal density of NP-108 is 1.4552, which indicates that it is not a pure polymorphic structure.

| Mannitol Crystalline Density Density $gm/cm^{-3}$ | Beta 1.49 | Alpha 1.468 |
|---|---|---|
| *Artur Burger, Jan-Olav Henck, Silvia Hetz, Judith Rollinger, Andrea Weissnicht, Hemma Stottner. *Journal of Pharmaceutical Science* 89.4 (2000): 457-468. | | |

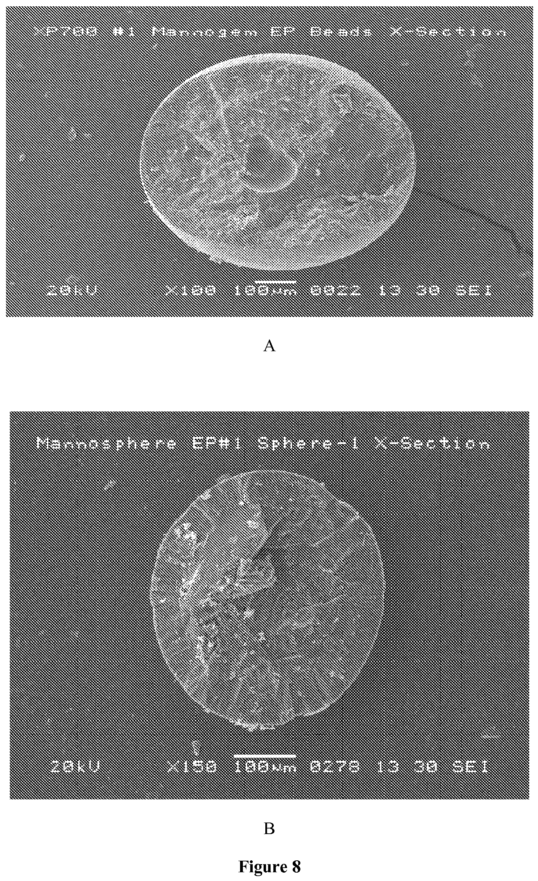

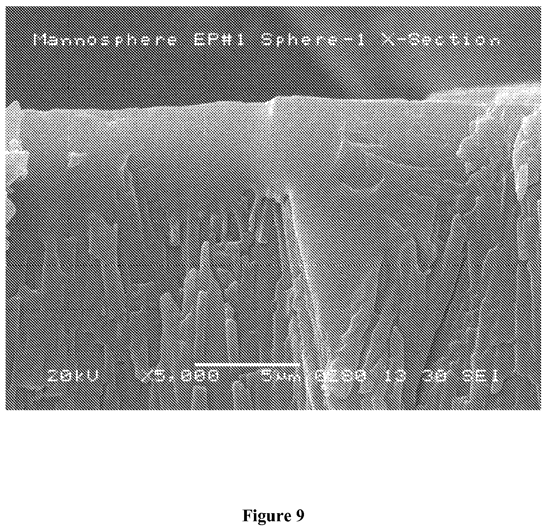

Scanning electron microscopy (SEM) was performed on sectioned mannitol microspheres to examine the interior structure of the microspheres. Figure 8 A and B are micrographs of the SEMs showing that the mannitol microsphere is solid and lacks interior voids. Figure 9 is a micrograph of the SEM shows that there is present a thin layered surface area. Figure 9 also shows vertical or radially rising, very tightly packed crystal formations that are underneath this upper layered surface structure. This upper layer of tightly packed crystal plates with ridges form roughened shallow ridges < 2 $\mu$m in height that allow a film polymer to grip to the surface with minimal loss into depression depths during the early stages of coating. The extremely shallow depth of the ridges creates a surface that allows for a minimum loss of film material deposits needed for a functional film. This very dense inner layer and the solid center of the bead allow the skeletal density of the microsphere to approach the true density reported for Alpha Mannitol, and allow for a very narrow control of particle density.

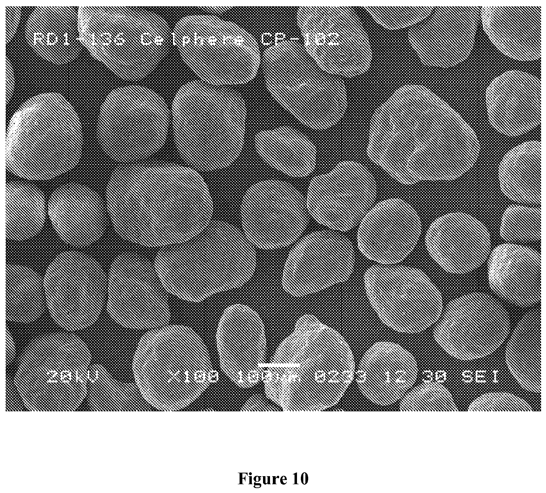

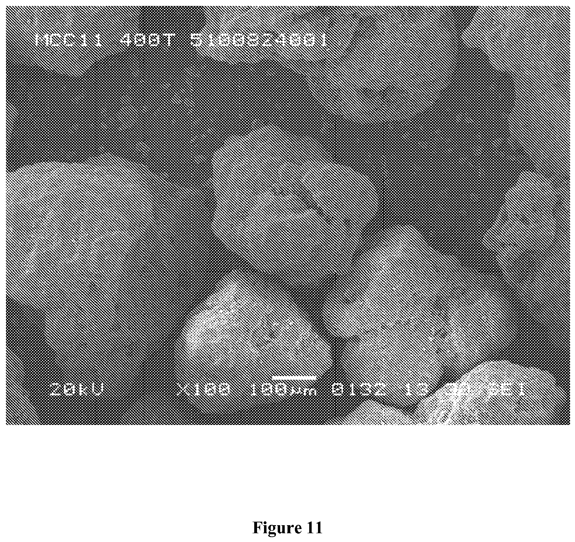

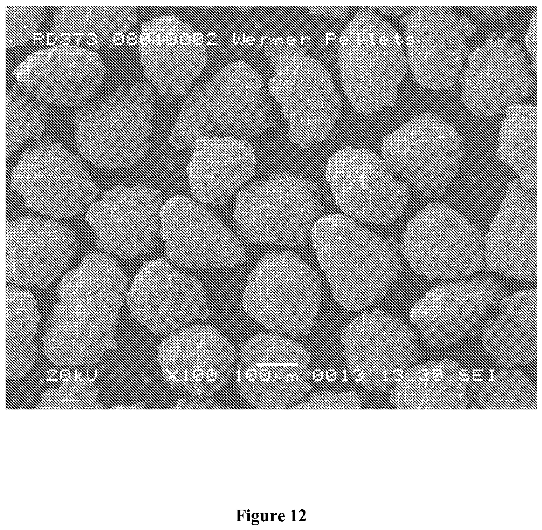

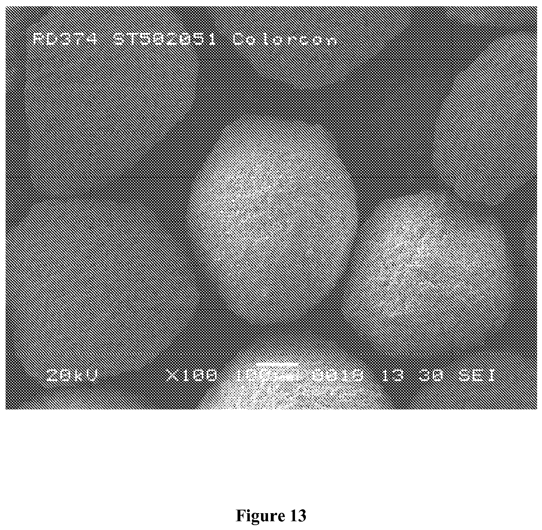

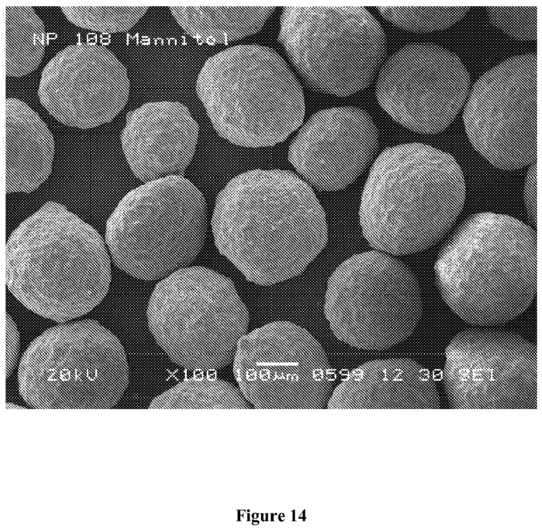

SEM was also performed on current commercial microspheres/beads: Celphere CP-102 microcrystalline cellulose beads (Asahi Kasei Corporation, Tokyo, Japan), MCell 400 mannitol beads (Pharmatrans Sanaq AG, Allschwil, Switzerland), Pharm-a-Sphere™ Neutral Pellets (Hanns G. Werner GmbH, Tornesch, Germany), SureSpheres® sugar/starch spheres (Colorcon, West Point, PA), Nonpareil-108 (NP-108) mannitol beads (Freund Industrial Co., Japan). Figure 10 is a micrograph of SEM of Celphere CP-102 microcrystalline cellulose beads, which have a smooth polymer type surface with some risers and convexed indentations present but limited cracks and fissures. The common watermelon or potato shape is apparent which would cause these particles to wabble in flow versus roll and also then to segregate on a shape basis. Figure 11 is a micrograph of SEM of MCell 400 mannitol beads, which shows the imperfect spherical nature of the beads, as well as the presence of convexity and lack of solidity caused by very deep fissures and risers. Also the bead structure is a fusion of multiple particles in a granular form verses a singular crystal body. Figure 12 is a micrograph of SEM of Pharm-a-Sphere neutral pellets, which shows a lack of the deep crevices seen in the MCell beads but shows what appears to be a single grown particle without appearance of agglomeration. Figure 13 is a micrograph of SEM of SureSpheres, which shows a non-spherical appearance, solid body and a lack of crevices. Figure 14 is a micrograph of SEM of NP-108 beads. The rolling motion used to make the NP-108 beads is obvious in the linear risers that curve in a spiral manner on the surface of the bead. The shape is round to cantaloupe to egg-shaped. Lack watermelon type shapes. Surface has crater type dish depression rather than ridges. The edges of these ridges are smoothed out based on the process of wet rolling during the manufacturing. Based on non-spherical shapes created in the process of manufacture, which appears to be a rolling/tumbling process this material will tend to segregate during the coating process. Presence of the cantaloupe to egg-shapes will create both shape motion variation and shape and size segregation during in the coating process. Mannosphere microspheres of the present invention are substantially all spherical and will not have the shape motion variation or shape segregation issue as great as NP-108 beads. For Mannospheres, microsphere size would control particle distribution during coating process as shape is more spherical.

**Circularity**

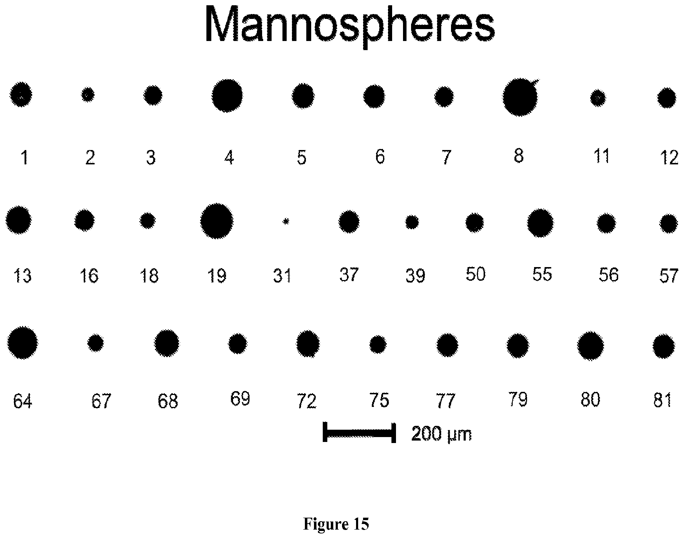

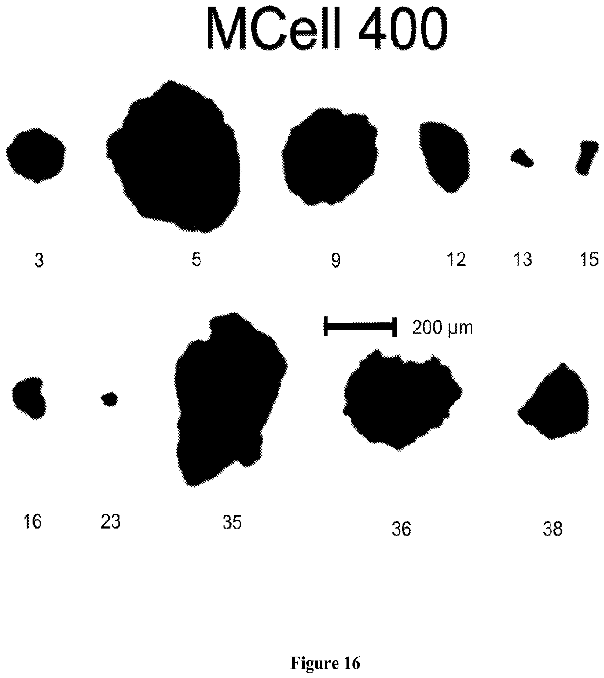

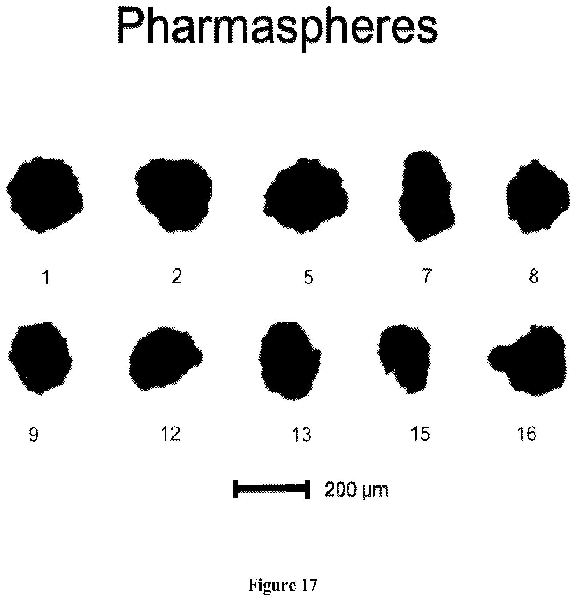

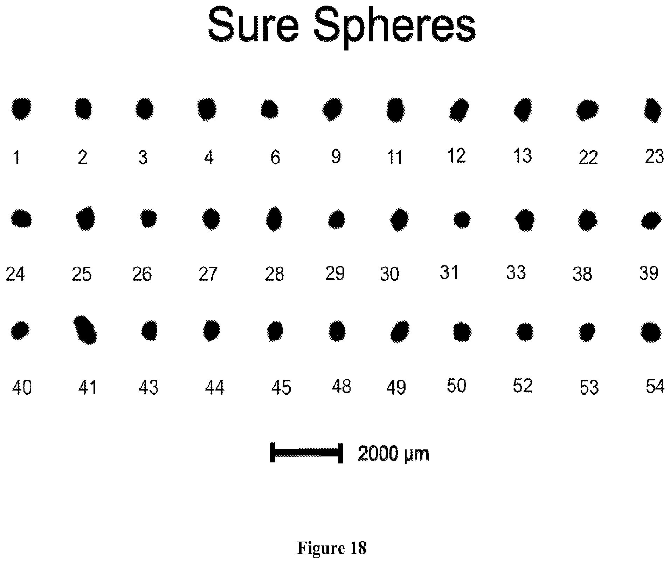

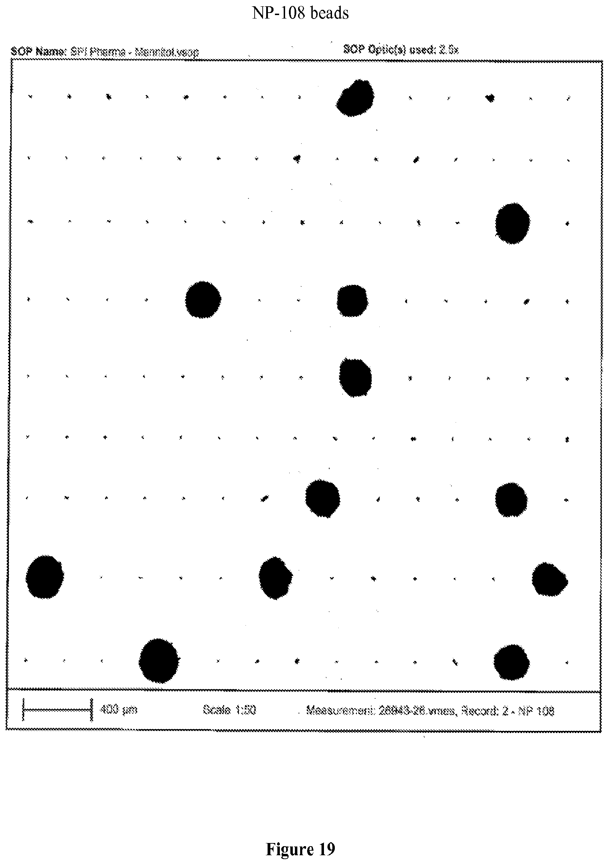

Characterization of the sphericity or circularity of microspheres was conducted by Particle Technology Labs (Downers Grove, IL, USA). Automated microscopy and image analysis techniques (Malvern Morphologi G3S automated particle image analysis system, Malvern Instruments Inc., USA) were used to characterize the morphology of microspheres of the present invention, and to compare to current commercially available microspheres, and to calculate mean circularity, aspect ratio, convexity and solidity of each. Figures 15-19 are the images generated for each. The number below the microsphere in Figures 15-18 is the random selection of microsphere the instrument used to print the silhouettes. Figure 15 is an image of exemplary mannitol microspheres of the present invention. Note the only thing seen out of round in the silhouette picture is particles with point appendages/attachments called twinings. These types of twining particles can be prevented or removed after manufacture to create a product of perfect spherical shape. Figure 16 is an image of MCell 400 mannitol beads (Pharmatrans Sanaq AG, Allschwil, Switzerland), which shows their non-perfect spherical nature, the presence of risers and of convexed surfaces. Figure 17 is an image of Pharm-a-Sphere™ Neutral Pellets (Hanns G. Werner GmbH, Tornesch, Germany), which shows they are non-perfect spheres with risers. Figure 18 is an image of SureSpheres® sugar/starch spheres (Colorcon, West Point, PA), which shows their non-spherical appearance. Figure 19 is an image of Nonpareil-108 mannitol beads (Freund Industrial Co., Ltd., Tokyo, Japan), which shows a large amount of finer particles, and they appear to have crevices and risers on the surface. Many of the beads/particles lack spherical shape and generate a potato shape, which is characteristic of the granulation and growth by layering process.

The circularity of each of the various microspheres was determined. Circularity is a measurement of the calculated peripheral length of a circle of the same silhouetted area of a particle's blocking a light source/ the particle's actual peripheral length with values in the range from 0 - 1. A perfect circle has circularity of roundness 1.0, while a needle-shaped object has roundness close to 0. Table 6 shows the circularity of various shapes (Image Analysis: Evaluating Particle Shape by Horiba Particles on July 07, 2011 by Jeff Bodycomb www.horiba.com).

Table 6: Circularity of various shapes

| Shape | | | | | | |
|---|---|---|---|---|---|---|
| Circularity | 1.0 | 0.886 | 0.777 | 0.660 | 0.509 | 0.4 |

The circularity- is typically determined using the equation:

$$Circularity = 2(\pi\,area)^{0.5}/P$$

where A is the measured area and P is the perimeter length of the microspheres. Circularity is calculated in accordance with International Organization for Standardization (ISO) 9276-6 (2008).

## Circularity

Periphery of a circle of similar area/ real periphery
(Total # of pixels as area in equation/actual count of pixels on periphery)

Area
14 *3 =42
13*7 =91
8
5  or total of 144 pixels

$$Sphericity = \frac{2\sqrt{\Pi * Pixel\_Area}}{P_{real}} = \frac{42.5}{48} = .886$$

Periphery
8+2+1+9+1+8+4+2+3+4+3+1+2= 48

In this depiction, the 0.886 circularity relates to the shape of a square or in three dimensions a cube and indicates the particle has sharper point or corners in its surface structure and would tumble verses roll. Tumbling is damaging to coating and causes segregation on shape and sharp edges cause issues in both coating stress and coating distribution, resulting in uneven coating thickness and coat cracking. Sharp edges on the bead surface can also break off and become incorporated in the coating and cause cracking and early release issues. Sharp edges can also add stress to the coating as it dries and cause the coating to crack. The presence of cracking can lead to the need to use more plasticizer which causes the coated particle to be tackier. Table 7 shows the circularity of the various microspheres tested.

Table 7: Circularity of various microspheres

| Material | Maker | Circularity% > 0.99 | Circularity % <0.99 >0.98 | Circularity % <0.98 >0.97 | Circularity % <0.97 >0.96 |
|---|---|---|---|---|---|
| Mannospheres | SPI Pharma | 74% | 9.42% | 4.97% | 4.93% |
| SureSpheres 20/25 | Colorcon | 0% | 0.36% | 14.34% | 27.06% |
| MCell 400 T | Pharmatrans Sanaq AG | 0% | 0% | 0.43% | 0.97% |
| Pharm-a-Spheres | Hanns Werner GmbH | 0% | 0% | 0% | 0.53% |
| NP-108 | Freund Industrial Co. | 0% | 32.85% | 32.85% | 13.97% |

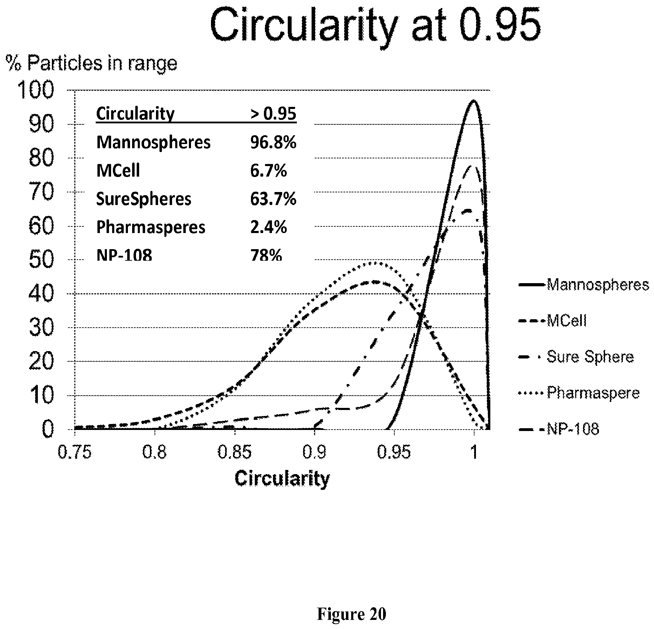

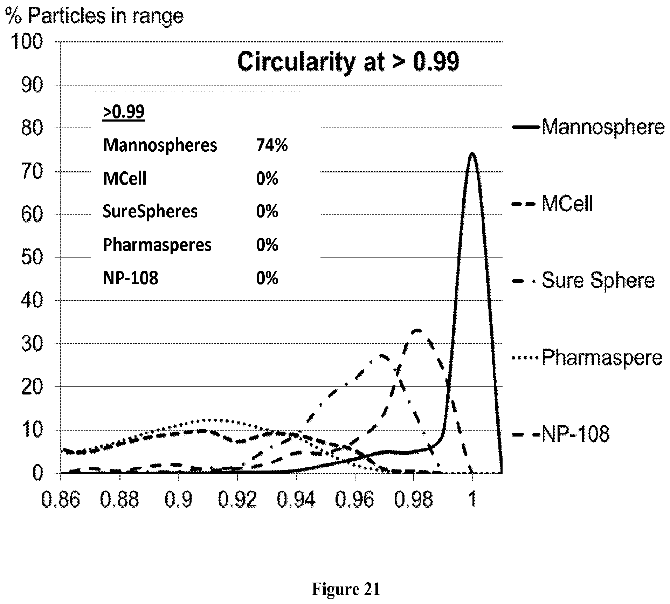

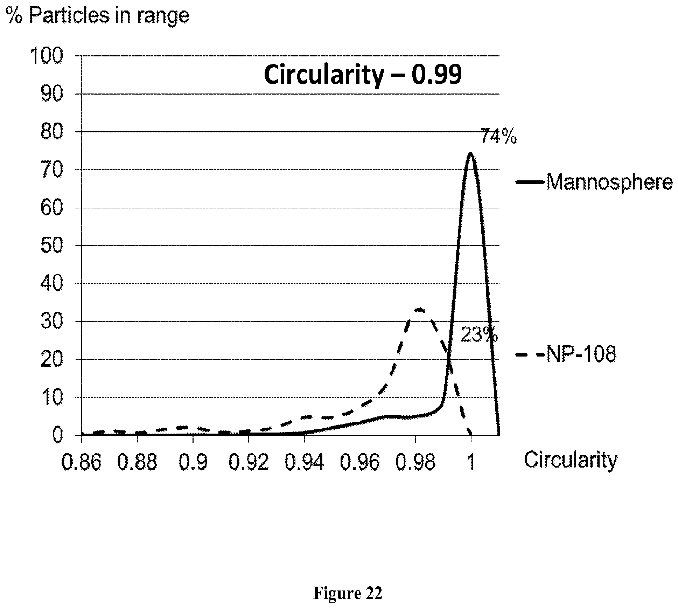

Figure 20 is a graph of the circularity at 0.95 of the various microspheres examined. Figure 21 is a graph of the circularity at 0.99 of the various microspheres examined. Figure 22 is a graph of the circularity at 0.99 of the microspheres of the present invention and the NP-108 (Freund Industrial Co., Japan). The results show that >95% of the mannitol microspheres of the present invention (Mannosphere) are perfect circles at greater than a circularity of 0.99, with perfection being >0.995. Also note the difference in distribution between the various commercial materials. The values below 0.9 mixed in with values above 0.95 will lead to a segregation issue based on the fact that some microspheres will tumble while others will roll. The data on circularity demonstrates that 96.8% of the mannitol microspheres of the present invention have a greater than 0.95 circularity rating, while 0% of the MCell microspheres have a circularity of greater than 0.985. Therefore they would be expected to tumble more than roll. Likewise, 0% of the Pharm-a-Spheres have a circularity of greater than 0.975, and therefore they would be expected to tumble and bounce more than roll.

Likewise, 0% of the Surespheres have a circularity of greater than 0.99 with 72.7% greater than 0.95, thus some of these beads will roll but some will tumble more than roll.

**Aspect Ratio**

The aspect ratio of each of the microspheres was also determined. Aspect ratio is defined as the ratio of the length of a sphere divided by the width, with the microspheres being considered circular (spherical) if the aspect ratio lies between 0.95 and 1.00. Table 8 shows the aspect ratios of various shapes (Image Analysis: Evaluating Particle Shape by Horiba Particles on July 07, 2011 by Jeff Bodycomb www.horiba.com). The aspect ratio is sensitive to how isometric the particle is. Particles with a high aspect ratio not only tumble but they tend to lodge in pore spaces in the coating bed and bounce as they tumble. This is a measure of rod, plate or needle-like characteristics of a particle.

Table 8: Aspect ratio of various shapes

| Shape | | | | | | |
|---|---|---|---|---|---|---|
| Circularity | 1.0 | 0.886 | 0.777 | 0.660 | 0.509 | 0.4 |
| Aspect Factor | 1.0 | 1.0 | 1.0 | 0.25 | 0.10 | 0.05 |
| Aspect Ratio | 1:1 | 1:1 | 1:1 | 1:4 | 1:10 | 1:20 |

## Aspect Ratio

Shortest Feret diameter/longest Feret diameter

12/14 = 0.857

Aspect Ratio = L / T

Aspect ratio is the ratio of the shortest diameter of particle to the longest diameter of a particle. Aspect ratio is calculated in accordance with International Organization for Standardization (ISO) 9276-6 (2008). Feret measured diameters as parallel lines brought in to touch particle at any angle. Thus it is the shortest separation of these lines divide by the longest separation.

Aspect Ratio is peak height but not crevice biased. The closer the aspect ratio is to 1 the more free rolling, less tumbling, mechanically interlocking and bouncing is a particle will it flows during the process of coating.

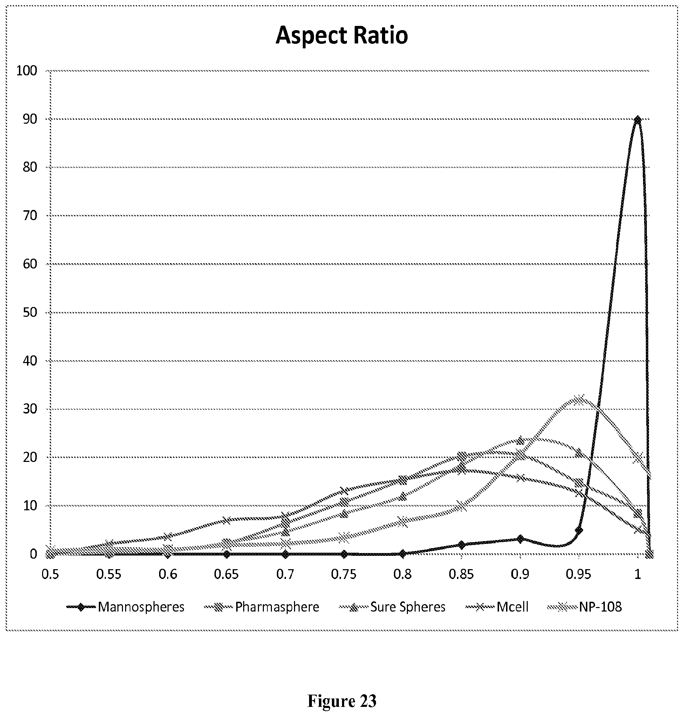

Table 9 and Table 10 show the aspect ratios of the microspheres tested and Figure 23 is a graph of the aspect ratios of the various microspheres tested. The results show a large disparity between other commercially available beads/microspheres and the microspheres of the present invention. With 89.8% of the microspheres of the present invention (Mannospheres) greater than 0.95 in aspect ratio and all the other beads less than 12% have aspect ratio of greater than 0.95. Also note the distribution of aspect ratios in the samples. The closer the aspect ratio is to 1.0, the more free rolling, less tumbling, mechanically inter locking and bouncing is a particle will it flows during the process of coating. Note the large disparity between other commercially available beads and the beads of this invention. Motion patterns change based on aspect ratio thus a narrow distribution of aspect ratio tend to flow the same with less segregating. A broad aspect shape range tends to segregate based on its motion patterns being different. Thus the broader the aspect ratio or factor the greater the segregation risk. Coating uniformity requires control of the surface being coated in the spray pattern called the spray flux, appearance of the microsphere in the area of the spray. Mixing of spherical particles of equal size with other shapes such as watermelon, oblong, flakes, and/or rods will cause changes in flux rate of a microsphere. This can occur by submersion of the particles under the bed below the surface being sprayed on, movement of the particles based on its shape more rapidly/slowly thought the spray zone or movement of the particle based on its shape alone into regions/area where spray is not being applied or applied as fast.

Table 9: Aspect ratio of the microspheres tested

| Material | Maker | Lot | Aspect ratio % > 0.95 | Aspect ratio % <0.95 >0.90 | Aspect ratio % <0.90 >0.85 | Aspect ratio % <0.85 >0.80 |
|---|---|---|---|---|---|---|
| Mannospheres | SPI Pharma | 10/7/11-4 | 89.83% | 5.00% | 3.15% | 1.92% |
| SureSpheres 20/25 | Colorcon | ST502051 | 8.41% | 21.11% | 23.61% | 18.43% |
| MCell 400 T | Pharmatrans Sanaq AG | 5100824001 | 5.14% | 12.65% | 15.76% | 17.26% |
| Pharm-a-Spheres | Hanns Werner GmbH | 08010002 | 8.47% | 14.74% | 20.46% | 20.25% |
| NP-108 | Freund Corporation | 109C-26 | 19.93% | 31.88% | 20.65% | 9.96% |

Table 10: Aspect ratio of the microspheres tested

| Material | Maker | Aspect ratio % > 0.99 | Aspect ratio % <0.99 >0.98 | Aspect ratio % <0.98 >0.97 | Aspect ratio % <0.97 >0.96 | Aspect ratio % <0.96 >0.95 | Aspect ratio % <0.95 |
|---|---|---|---|---|---|---|---|
| Mannospheres | SPI Pharma | 64.45% | 16.10% | 4.55% | 2.60% | 1.82% | 10.48% |
| SureSpheres 20/25 | Colorcon | 1.07% | 1.43% | 1.43% | 1.97% | 2.50% | 91.59% |
| MCell 400 T | Pharmatrans Sanaq AG | 0.75% | 0.64% | 0.96% | 1.07% | 1.71% | 94.86% |
| Pharmaspheres | Hanns Werner GmbH | 1.67% | 1.46% | 1.48% | 1.74% | 2.13% | 91.53% |
| NP-108 | Freund Industrial Co. | 2.90% | 1.63% | 3.08% | 5.07% | 7.25% | 80.07% |

**Solidity**

The microspheres of the present invention and current commercial microspheres/beads were examined for solidity. Solidity looks for missing areas caused by risers or indentations in the surface of the microsphere or particle. In order to determine the solidity, a cord is wrapped around the microsphere to approximate the area of the microsphere or particle without convex (indented areas) due to crevices and risers off the surface. The area of the microsphere or particle is exactly measured as the shadow of the image of the microsphere or particle in a light path. The area of the microsphere or particle is then divided by the area inside the cord stretched over the microspheres' or particles' outer surface. Solidity is calculated in accordance with International Organization for Standardization (ISO) 9276-6 (2008).

A sphere has a solidity of 1. A cube, a triangle (pyramid) or a rod would also have a solidity of 1. Although a cube and a pyramid have corners/edges, they do not have surface risers or crevices. Any surface indentations or surface bumps would add to the area inside the cord. Thus solidity as a factor is then related to the area associated with the convexity area of the microsphere as area lost ratio to solidity. A surface without convexness can be directly coated in layers. Each layer at the start is the base layer and grows uniformly in thickness. Issues with convexed areas is removing the air, getting the film in the tighter space uniformly and building the layer in the space to the surface to allow for a uniform outer layer coating thickness. Extra time is spent, smaller droplets sizes of coating spray may be required, more plasticizer needed to allow the film to bridge without cracking if the crevice isn't filled and extra coating material is used.

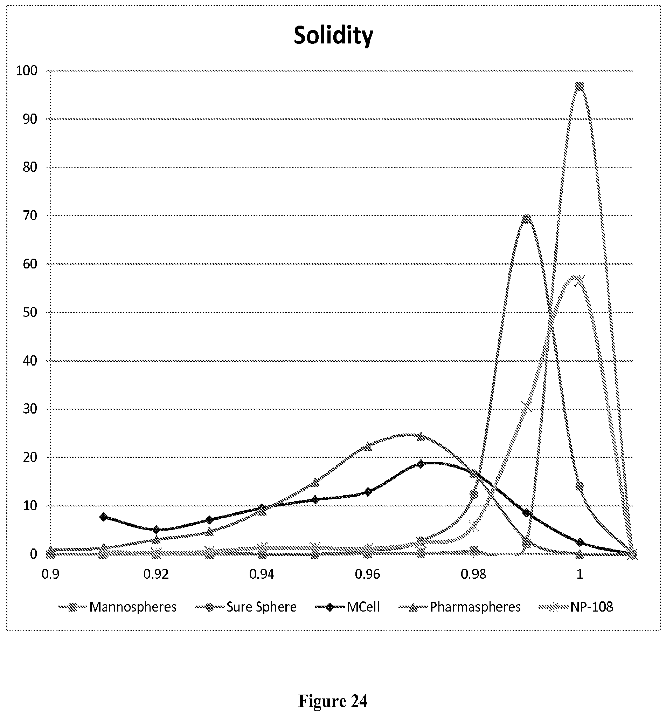

In three dimensions it is also related to the extra volume associated with this convexed space. Which either coating fills or bridges over creating coating stress, imperfections and variation in coating amount needed to obtain a functional coating. Table 11 shows the solidity of the microspheres tested and Figure 24 is a graph of the solidity of the various microspheres tested. The graph demonstrates the lack of solidity of the mannitol microspheres of the present invention (Mannosphere) and the narrow range of solidity in mannitol microsphere samples. Note the forecasted surface to be coated is consistent with a smoothed surface without risers or crevices for 96% of the particles at a solidity factor of 0.99. A narrow range of solidity for the mannitol microspheres also aides in coating thickness consistency and direct coating layering. Maintaining the film surface contour. It is apparent in the SEMs the crevices in the MCell 400 beads as well as the separation in the NP-108 beads made by a similar granulation route would require additional coating material to fill these spaces. The SureSpheres and the Pharm-a-Sphere beads would loss coating material into the contour of the risers.

Table 11: Solidity of the microspheres tested

| Material | Maker | Lot | Solidity % > 0.99 | Solidity % <0.99 >.98 | Solidity % <0.98 >.97 | Solidity % <0.97 >.96 |
|---|---|---|---|---|---|---|
| Mannospheres | SPI Pharma | 10/7/11-4 | 96.7% | 2.29% | 0.72% | 0.17% |
| SureSpheres 20/25 | Colorcon | ST502051 | 14.0% | 69.35% | 12.37% | 2.69% |
| MCell 400 T | Pharmatrans Sanaq AG | 5100824001 | 2.5% | 8.57% | 16.72% | 18.65% |
| Pharm-a-Spheres | Hanns Werner GmbH | 08010002 | 0% | 2.96% | 16.71% | 24.39% |
| NP-108 | Freund Industrial Co. | 109C-26 | 56.5% | 30.43% | 5.80% | 2.36% |

**Convexity**

Convexity

- Area of the particle vs Area of Particle plus missing area connected

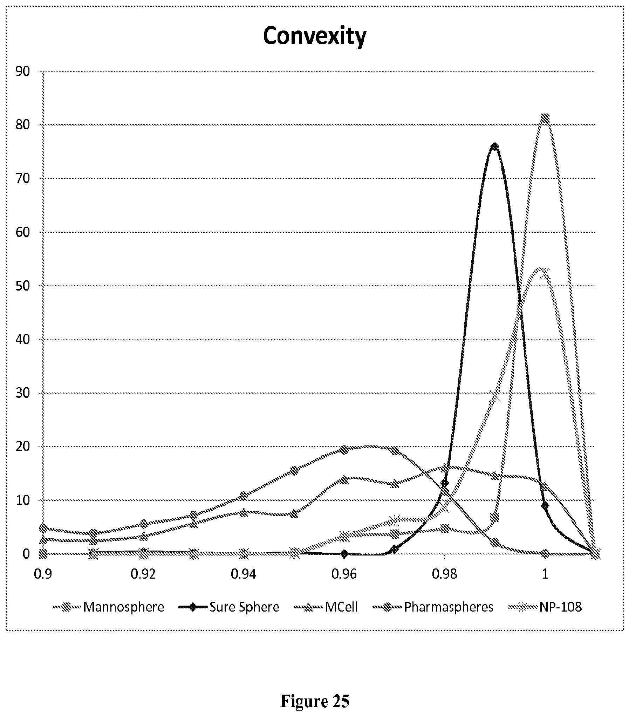

The microspheres of the present invention and current commercial microspheres/beads were also examined for convexity. Convexity is similar to solidity but focuses more on surface smoothness. Here the most accurate measurement is the periphery of the particle. What the approximation is in this index is the cord length that is drawn surrounding the particle which is the same cord length as in the solidity measurement. If the surface is perfectly smooth and without crevices or risers the convexity will be equal to one. Convexity is calculated in accordance with International Organization for Standardization (ISO) 9276-6 (2008). Table 12 shows the convexity of the microspheres tested and Figure 25 is a graph of the convexity of the various microspheres tested. Note the forecasted surface to be coated is consistent with a smoothed surface without risers or crevices for 81 % of the particles at a convexity of 0.99. It is apparent in the table that the effective surface area for coating is lost in area difference between the area of a perfectly spherical shape and into either crevice or riser imperfections. The difference at a scale of comparison at 0.99 is substantial.

Table 12: Convexity of the microspheres tested

| Material | Maker | Lot | Convexity % > 0.99 | Convexity % <0.99 >0.98 | Convexity % <0.98 >0.97 | Convexity % <0.97 >0.96 |
|---|---|---|---|---|---|---|
| Mannospheres | SPI Pharma | 10/7/11-4 | 81.2% | 6.82% | 4.73% | 3.73% |
| SureSpheres 20/25 | Colorcon | ST502051 | 9.0% | 75.99% | 13.26% | 0.90% |
| MCell 400 T | Pharmatrans Sanaq AG | 5100824001 | 12.6% | 14.68% | 16.08% | 13.18% |
| Pharm-a-Spheres | Hanns Werner GmbH | 08010002 | 2.3% | 2.11% | 11.64% | 19.19% |
| NP-108 | Freund Industrial Co. | 109C-26 | 52.4% | 29.35% | 8.70% | 6.16% |

**Surface Area/Porosity**

The microspheres of the present invention and current commercial microspheres/beads were also examined for determination of surface area and porosity. Surface area measurements of the samples were performed using a Tristar II 3020 surface area analyzer made by Micromeritics (Malvern, PA). The analysis gas was nitrogen; the analysis temperature was 77.4 K, cold free space was 40.1922 to 41.4298 $cm^3$ measured and the warm free space was 13.1291 to 13.4346 $cm^3$ measured. The equilibration interval was 20 seconds, and the sample density was assumed to be 1.000 $gm/cm^3$. The mass of the samples was accurately weighed to approximately 2.5 gms. Single point BET was run at a relative pressure of 0.15 to 0.20 and extrapolated to zero. Multipoint was done at least two additional relative pressure points below the single point measurement relative pressure. Tristar II 3020 V1.04 software was used to calculate the single point and multipoint BJH adsorbed and desorbed surface area.

The pore volume measurements were performed on the same instrument used for surface area measurement at relative pressure up to 1.0. A sample accurately weight of approximately 0.5 gms was used. Ten or more relative pressure from 0.01 to 1.0 was used. Tristar II 3020 VI.04 software was used to calculate the pore volume. Table 13 shows the surface area and porosity of the various microspheres examined. Data comparing surface area and pore volume clearly shows the Mannosphere microspheres of the present invention have less surface area than the Sure-Sphere and Pharm-a-Sphere beads in single point and in both the BJH multipoint adsorption and desorption methods. This lack of pore volume and lower surface area per gram is due to Mannosphere microspheres' shape being more spherical, and the lack of internal surface compared to the Sure-Sphere or Pharm-a-Sphere beads or is due to bead size.

Table 13: Surface area and Porosity

| Sample | Single point BET Surface Area (M2/g) | BJH Adsorption Surface Area (M2/g) | BJH Desorption Surface Area (M2/g) | BJH Adsorption Pore Volume (CM3/gm) | BJH Desorption Pore Volume (CM3/g) |
|---|---|---|---|---|---|
| Mannosphere Beads | 0.04 | 0.005 | 0.0498 | 0.000274 | 0.000005 |
| SureSphere Beads | 0.26 | 0.167 | 0.1844 | 0.001051 | 0.000209 |
| Pharm-a-Sphere Beads | 0.22 | 0.132 | 0.1433 | 0.00697 | 0.000152 |

Particle size for each of the microsphere samples was measured by laser particle size analysis using a Microtrac S350 made by Microtrac Corporation (Montgomeryville, PA). Approximately a one gram sample was used for the test using the Turbotrac dry feeder at an energy setting of 10 and a range of 0.687 to 995.6 $\mu$m and a 10 second run time. The samples particle size and the samples projected surface area as a perfect was calculated by the Microtrac Flex version 10.4 program supplied with the analyzer. Data on particle size is listed in the Table 14. All samples were assigned a 1.0 gm/ ml density to convert volume values to grams.

Table 14: Particle size and calculated surface area for various microspheres

| Sample | Calculated Surface from Size data M$^2$/gm | d(0.1) um | d(0.5) um | d(0.9) um |
|---|---|---|---|---|
| Mannitol Microsphere EP | 0.0273 | 133 | 259 | 373 |
| Suresphere Beads | 0.0145 | 329 | 413 | 608 |
| Pharmasphere Beads | 0.035 | 135 | 175 | 233 |

Table 15 compares the size estimate of surface area in the multiparticulate sample to the actual surface area measured by nitrogen gas adsorption. Mannosphere microspheres show a much closer prediction of size of beads to surface area of the bead, which is only going to be typical of true spherical beads lacking internal porosity. The Mannosphere microspheres are smaller in particle size (259 $\mu$m) to Sure-Spheres beads (413 $\mu$m). Thus from microsphere size data, Sure-Sphere beads are expected to have a lower surface area. The actual surface area in Sure-Sphere beads is (0.26/.04)= 6.5 times more than Mannosphere microspheres. Similarly the Pharm-a-Sphere beads are smaller (175 $\mu$m), yet they have 5.5 times more surface area than predicted. The actual to predicted surface area shows how much closer Mannosphere microspheres have a size predicted surface area for coating.

This data along with the pore volume measurements shows the substantial lack of internal surface as well as the closer match to prefect sphericity by the microspheres of this invention compared to standard mass marketed microspheres or beads. This tighter microsphere size to surface control allows for a much more accurate use of size to control both amount of surface coating per batch but control the surface location as more on the outer surface of the bead versus interior to the microsphere.

Table 15: Actual to Predicted Surface Areas of various microspheres

| Sample | Asz, Calculated Surface from Size data M$^2$/gm | A$_{act}$, Single point BET Surface Area (M$^2$/gm) | Actual to Predicted Surface Area (A$_{act}$/A$_{sz}$) |
|---|---|---|---|
| Mannitol Microspheres EP | 0.0273 | 0.04 | 1.5 |
| Sure-Sphere Beads (Colorcon) | 0.0145 | 0.26 | 18 |
| Pharm-a-Sphere Beads (Hanns G Werner) | 0.035 | 0.22 | 6.3 |

**Dissolution**

The microspheres of the present invention and current commercial microspheres/beads were also examined for determination of dissolution. The dissolution of microsphere samples was performed in 20 mL glass vial with cap. (Fisher Scientific, Hampton, NH) 10 mL of deionized water was added to an accurate weight of approximately 500 mg of microspheres. The vials were place into the holder of the Rotatest Ping-Pong model 51500-10 reciprocating shaker (Cole Parmer, Vernon Hills, IL) and its platform rotated at 300 rpm. After shaking for test time, the samples were filtered through pre-weighed 0.45 micron filter paper (Nylon Membrane filters, 0.45 micron, 47 mm dia, Cat # 7404004, Whatman (Fisher Scientific, Hampton, NH)). The filter paper was dried in a conventional oven at 60 °C and then placed in a suitably sized glass desiccator (Fisher Scientific, Hampton, NH) using fresh drierite (Sigma Aldridge, St. Louis, Mo). Once cooled the filter paper was re-weighed to calculate the weight change in mg. Table 16 shows the dissolution of various microspheres. The data shows the limited amount of < 0.5% undissolved at both 5 and 10 minutes of testing compared to NP-108 microspheres at 2.0% in 5 minutes and 0.92% in 15 minutes, and Sure-Sphere and Pharm-a-Sphere beads with > 10% undissolved even at 15 minutes of shaking. 100% dissolution or solubility is often desirable in situations in which very low dose actives are used in coatings to obtain complete recovery of actives.

Table 16: Dissolution of various microspheres

| Dissolution (0.5 g sample in 10 ml Water On Shaker @ 300 rpm) | | |
|---|---|---|
| Sample | Weight of residue on filter paper @ | |
| | 5 mins | 15 mins |
| Mannitol micro spheres EP | 1.9 mg or 0.38% | 1.9 mg or 0.38% |
| NP-108 (Freund Industrial Co.) | 10.2 mg or 2.04% | 4.9 mg or 0.92% |
| Pharm-a-Sphere (Hanns G Werner) | 61.1 mg or 12.2% | 60.7 or 12.1% |
| Sure-Spheres (Colorcon) | 100 mg or 20% | 98.1 or 19.6% |

**Oil Adsorption**

The microspheres of the present invention and current commercial microspheres/beads were also examined for oil adsorption. 1.00 g of each microsphere sample was accurately weighed. The sample was added to a mortar and pestle and 0.02 g of Light Mineral Oil, USP/NF (Sigma Aldrige, St. Louis, MO) was added drop-wise (Transfer pipet, disposable, standard, polyethylene, one-piece, single squeeze draws up to 3.2 mL, length 5.875 in., capacity 7.7 mL;Fisher Scientific, Hampton, NH). The oil was thoroughly incorporated into the sample by lightly mixing, but not crushing in the mortar and pestle (Mortar and Pestle: Coors, USA. Cat # 60319, Fisher Scientific, Hampton, NH) for 3 minutes. The samples were visually observed to determine is the sample was free flowing or sticky/agglomerated. Table 17 shows the oil adsorption for the various microspheres. None of the samples tested was able to adsorb 2% oil and remain as free flowing beads. All of these beads are constructed to have minimum oil adsorption capacity.

Table 17: Oil Adsorption of various microspheres

| Oil Adsorption Capacity | | |
|---|---|---|
| | Observation @ | |
| Sample (1.00 g) | 0.1 g Mineral Oil | 0.02 g Mineral Oil |
| Mannitol Microspheres EP | Sticky powder | Sticky powder |
| NP-108 (Freund Industrial Co.) | Sticky powder | Sticky powder |
| Pharm-a-Sphere (Hanns G Werner) | Sticky powder | Sticky powder |
| Sure-Spheres (Colorcon) | Sticky powder | Sticky powder |

Example 3

Erythritol EP (Baolingbao Biology Co., LTD., China) was added to a Tornado Spin Disc (Gold Metal Cincinnati Ohio). The units spin head was heated to ~160°C while spinning at 3400 RPM and made microspheres with a PSD of d(0.1) = 131 $\mu$m, a d(0.5) = 262 $\mu$m and a d(0.9) = 371 $\mu$m. The size distribution ratio is 2.8 to 1 for this run. The DSC of these beads shows a single and sharp melt peak at 121.6°C with a heat of fusion of 273.1 J/g. A pure crystalline erythritol melt range of 199°C to 121°C is expected and thus crystal structure of formed microsphere is a standard and highest energy erythritol polymorph.

In the following embodiments of the invention are summarized:

1. A composition comprising microspheres, wherein the microspheres have a circularity greater than 0.98, an aspect ratio greater than 0.98 and surface ridges less than 1 $\mu$m in height.

2. The composition of item 1, wherein the microspheres have a circularity greater than 0.99 and an aspect ratio greater than 0.99.

3. The composition of item 1, wherein the microspheres have a mean particle size from about 10 $\mu$m to about 500 $\mu$m.

4. The composition of item 3, wherein the microspheres have a mean particle size from about 10 $\mu$m to about 20 $\mu$m.

5. The composition of item 4, wherein the microspheres have a moisture content of 0.5 % or less.

6. The composition of item 1, wherein the microspheres have a particle size distribution of 2.8 or less.

7. The composition of item 6, wherein the microspheres have a particle size distribution of 2.0 or less.

8. The composition of item 7, wherein the microspheres have a particle size distribution of 1.5 or less.

9. The composition of item 8, wherein the microspheres have a particle size distribution of 1.0.

10. The composition of item 1, wherein the microspheres have a skeletal density from about 1.4595 g/cc to about 1.4651 g/cc.

11. The composition of item 1, wherein the microspheres have a moisture content of 0.5 % or less.

12. The composition of item 11, wherein the microspheres have a moisture content of 0. 1% or less.

13. The composition of item 11, wherein the microspheres have a moisture content of 0.0%.

14. The composition of item 1, wherein the microspheres are water soluble.

15. The composition of item 1, wherein the microspheres comprise a single material.

16. The composition of item 15, wherein the single material is mannitol.

17. The composition of item 1, wherein the microspheres comprise a 100% crystalline core.

18. The composition of item 1, wherein the microspheres further comprise an active pharmaceutical ingredient.

19. The composition of item 1, wherein the microspheres comprise a polyol.

20. The composition of item 19, wherein the polyol is mannitol.

21. The composition of item 19, wherein the polyol is erythritol.

22. The composition of item 1, wherein the microspheres lacks porosity.

23. The composition of item 1, wherein the microspheres lack internal voids.

24. The composition of item 1, wherein the microspheres comprise one or more polyols.

25. The composition of item 24, wherein the one or more polyols is mannitol.

26. The composition of item 24, wherein the one or more polyols is mannitol and sorbitol.

27. A composition comprising microspheres, wherein the microspheres have a circularity greater than 0.98, an aspect ratio greater than 0.98 and skeletal density from about 1.4595 g/cc to about 1.4651 g/cc.

28. A pharmaceutical formulation comprising the composition of item 1.

29. The pharmaceutical formulation of item 28, further comprising an active pharmaceutical ingredient.

**Claims**

1.  A composition comprising microspheres, wherein the microspheres have a circularity greater than 0.98, an aspect ratio greater than 0.98, and skeletal density from about 1.4595 g/cc to about 1.4651 g/cc.

**2.** The composition of claim 1, wherein the microspheres have an average skeletal density of about 1.461g/cc.

**3.** The composition of claim 1, wherein the skeletal density of the microspheres is within about -10% and +10% of the skeletal density of the microspheres' material, within about -5% and +5% of the skeletal density of the microspheres' material, within about -1% and +1% of the skeletal density of the microspheres' material, within about -0.5% and +0.5% of the skeletal density of the microspheres' material, or within about -0.1% and +0.1% of the skeletal density of the microspheres' material.

**4.** The composition of claim 1, wherein the microspheres have a moisture gain at about 90% relative humidity of less than about 2.0% by weight, less than about 1.0% by weight, less than about 0.5% by weight, less than about 0.1% by weight, or about 0% by weight.

**5.** The composition of claim 1, wherein the microspheres have a moisture gain at about 60% relative humidity of less than about 1.0% by weight, less than about 0.5% by weight, less than about 0.1% by weight, less than about 0.05% by weight, or about 0% by weight.

**6.** The composition of claim 1, wherein the microspheres have a moisture content of about 2.0% or less, about 1.0% or less, about 0.5% or less, about 0.1% or less, or about 0.05% or less.

**7.** The composition of claim 1, wherein the microspheres have a mean particle size in diameter from about 10 $\mu$m to about 500 $\mu$m, a mean particle size from about 10 $\mu$m to about 20 $\mu$m, or a mean particle size from about 2 $\mu$m to about 10 $\mu$m.

**8.** The composition of claim 1, wherein the microspheres have a particle size distribution (d(0.9)/d(0.1)) of about 2.8 or less, about 2.0 or less, about 1.5 or less, or about 1.2 or less.

**9.** The composition of claim 1, wherein the microspheres are water soluble.

**10.** The composition of claim 1, wherein the microspheres comprise a core material, and the core material comprises one or more polyols.

**11.** The composition of claim 10, wherein the microspheres comprise a single core material, wherein the core material comprises a polyol.

**12.** The composition of claim 11, wherein the core material is mannitol or sorbitol.

**13.** The composition of claim 1, wherein the microspheres lack internal porosity.

**14.** The composition of claim 1, wherein the microspheres lack internal voids.

**15.** A pharmaceutical composition comprising the composition of claim 1 and an active pharmaceutical ingredient.

Figure 1

Figure 2

Figure 3

**Figure 4**

**Figure 5**

**Figure 6A**

Figure 6B

Figure 6C

EP 3 583 935 A1

Figure 7

39

A

B

Figure 8

Figure 9

Figure 10

Figure 11

Figure 12

Figure 13

**Figure 14**

Mannospheres

1  2  3  4  5  6  7  8  11  12

13  16  18  19  31  37  39  50  55  56  57

64  67  68  69  72  75  77  79  80  81

200 µm

Figure 15

Figure 16

Figure 17

# Sure Spheres

| 1 | 2 | 3 | 4 | 6 | 9 | 11 | 12 | 13 | 22 | 23 |

| 24 | 25 | 26 | 27 | 28 | 29 | 30 | 31 | 33 | 38 | 39 |

| 40 | 41 | 43 | 44 | 45 | 48 | 49 | 50 | 52 | 53 | 54 |

⊢━━━━⊣ 2000 µm

Figure 18

NP-108 beads

Figure 19

# Circularity at 0.95

% Particles in range

| Circularity | > 0.95 |
|---|---|
| Mannospheres | 96.8% |
| MCell | 6.7% |
| SureSpheres | 63.7% |
| Pharmasperes | 2.4% |
| NP-108 | 78% |

Legend:
- —— Mannospheres
- ---MCell
- — · Sure Sphere
- ······Pharmaspere
- — -NP-108

Circularity (x-axis: 0.75, 0.8, 0.85, 0.9, 0.95, 1)

y-axis: 0, 10, 20, 30, 40, 50, 60, 70, 80, 90, 100

**Figure 20**

Figure 21

% Particles in range

**Circularity – 0.99**

74%

—Mannosphere

23%

- -NP-108

0.86  0.88  0.9  0.92  0.94  0.96  0.98  1   Circularity

**Figure 22**

Figure 23

Figure 24

Figure 25

Europäisches
Patentamt

European
Patent Office

Office européen
des brevets

# EUROPEAN SEARCH REPORT

Application Number

EP 19 16 3519

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHAMBI ET AL: "Solid lipid microparticles containing water-soluble compounds of different molecular mass: Production, characterisation and release profiles", FOOD RESEARCH INTERNATIONAL, ELSEVIER APPLIED SCIENCE, BARKING, GB, vol. 41, no. 3, 8 December 2007 (2007-12-08), pages 229-236, XP022511840, ISSN: 0963-9969, DOI: 10.1016/J.FOODRES.2007.11.012 * abstract * * item 2.5 * * figures 3,4 * | 1-15 | INV. A61K9/16 |
| X | LUCIANI A ET AL: "Solvent and melting induced microspheres sintering techniques: a comparative study of morphology and mechanical properties", JOURNAL OF MATERIALS SCIENCE: MATERIALS IN MEDICINE, KLUWER ACADEMIC PUBLISHERS, BO, vol. 22, no. 9, 24 July 2011 (2011-07-24), pages 2019-2028, XP019947843, ISSN: 1573-4838, DOI: 10.1007/S10856-011-4390-8 * page 2020, right-hand column * * figure 1 * | 1-15 | |

TECHNICAL FIELDS
SEARCHED (IPC)

A61K

The present search report has been drawn up for all claims

| Place of search | Date of completion of the search | Examiner |
|---|---|---|
| Munich | 18 November 2019 | Vázquez Lantes, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.............................................................................................
& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61638073 A

- US 61783603 A

**Non-patent literature cited in the description**

- Physician's Desk Reference. Thomson PDR, 2007